# EUROPEAN PATENT SPECIFICATION

(11) **EP 2 663 273 B1**
(45) Date of publication and mention of the grant of the patent: **04.04.2018**
(21) Application number: 12703878.4
(22) Date of filing: 13.01.2012
(51) Int. Cl.: A61F 13/15, A61F 13/56

(54) **PANT-TYPE DIAPER AND CORRESPONDING MANUFACTURING PROCESS AND APPARATUS**
HOSENARTIGE WINDEL SOWIE VERFAHREN UND VORRICHTUNG ZU IHRER HERSTELLUNG
COUCHE-CULOTTE DE TYPE CULOTTE ET PROCÉDÉ ET APPAREIL DE FABRICATION CORRESPONDANTS

(30) Priority: 13.01.2011 US 201113006159; 17.11.2011 US 201113299145
(43) Date of publication of application: 20.11.2013
(73) Proprietor: Fameccanica.Data S.p.A., 65129 Pescara (IT)
(72) Inventor: SABLONE, Gabriele, I-65016 Montesilvano (Pescara) (IT); PASQUALONI, Paolo, I-66020 San Giovanni Teatino (Chieti) (IT)
(74) Representative: Marchitelli, Mauro
(86) International application number: PCT/IB2012/000046
(87) International publication number: WO 2012/095739

(56) References cited:
- WO-A1-2010/008032
- US-A1- 2003 168 614
- US-A1- 2003 216 706

## Description

### CROSS REFERENCE TO RELATED APPLICATIONS

This application is a continuation-in-part of copending U.S. Patent Application Serial No. 13/299,145, filed on November 17, 2011, which is a continuation-in-part of copending U.S. Patent Application Serial No. 13/006,159, filed on January 13,2011.

### BACKGROUND

This disclosure relates to absorbent sanitary products. More particularly, this disclosure relates to refastenable absorbent sanitary products that can be worn like pants.

Over the last few years, there has emerged interest in diapers of the type commonly referred to as "training pants". When such a product is taken out of the pack, it has already a conformation that substantially resembles that of the pair of pants. It is put on by sliding it over the legs of the user according to criteria basically similar to the ones adopted for putting on pants.

A training pant typically includes a central body or chassis that contains an absorbent core which is designed to absorb the body fluids evacuated by the wearer. Side panels extend laterally from the chassis so as to *complete* the pant-like configuration of the product. The side panels are provided with homologous distal edges designed to be connected (pre-fastened) to one another to form lateral closure regions. In the most recent products the pre-fastened closure regions are intended to be refastenable, thus permitting the product - which is sold in a pre-fastened, closed pant-like condition - to be selectively opened at either side in order to check e.g. whether the product is soiled.

Documents such as U.S. Patents Nos. 6,761,711; 6,849,067; 6,645,190; or 7,534,237 are exemplary of arrangements of refastenable side closures.

Various patent documents such as U.S. Patents 6,514,187; 7,322,925; 7,335,150; 7,387,148 or EP-A-1 289 465, EP-A-2 289 466, or EP-A-1 284 700 are exemplary of processes and apparatus which may be applied to manufacturing products with refastenable side closures.

Despite the effectiveness of the results obtained, the various arrangements considered in the foregoing have an intrinsic disadvantage in that the associated manufacturing processes and apparatus are inevitably complex, expensive and exposed to criticalities in terms of reliability.

It would therefore be desirable to provide a solution dispensing with one of more or all of these drawbacks while leading to product structure retaining the advantages discussed in the foregoing. The claims are an integral part of the disclosure of the invention as provided herein.

### SUMMARY OF THE INVENTION

A method is provided for preparing a refastenable article in claim 1. Application WO 2010/008032 describes a method of manufacturing a refastenable article with hook and loop closure elements capable of preventing the loop elements engaged with hook elements from being curled up in a production step. A refastenable training pant is claimed in claim 6.

### BRIEF DESCRIPTION OF THE SEVERAL DRAWING VIEWS

The invention will now be described, by way of example only, with reference to the annexed representations, wherein:
FIG. 1 is an exploded perspective view of a refastenable article according to one or more embodiments of the present disclosure.
FIG. 2 is a plan view of a refastenable article in a flattened-out configuration in accordance with one or more embodiments of the present disclosure.
FIG. 3 is a partial schematic cross-section showing a refastenable article in accordance with one or more embodiments of the present disclosure.
FIG. 4 is a partial plan view of a refastenable article in a flattened-out configuration, showing details of a second breakable bond between the second folded portion and a second side panel in accordance with embodiments of the present disclosure.
FIG. 5 is a partial plan view of a refastenable article in a flattened-out configuration, showing details of a third and a fourth breakable bond between a first tabbed portion and a second tabbed portion, respectively, and the chassis in accordance with embodiments of the present disclosure.
FIG. 6 is an exploded perspective view of a refastenable article according to one or more embodiments of the present disclosure.
FIG. 7 is a plan view of a refastenable article in a flattened-out configuration in accordance with one or more embodiments of the present disclosure.
FIG. 8 is a partial plan view of a refastenable article in a flattened-out configuration, showing details of the folding of a back side panel in accordance with one or more embodiments of the present disclosure.
FIG. 9 is a partial schematic cross-section showing a refastenable article in accordance with one or more embodiments of the present disclosure.
FIG. 10 is a perspective view of the refastenable article depicted in FIGS. 1-2 and 6-7 in a configuration of use in accordance with one or more embodiments of the present disclosure.
FIG. 11 is an enlarged detail of the part indicated by the arrow V in FIG. 10 in accordance with one or more embodiments of the present disclosure.
FIG. 12 is a schematic representation of a method for manufacturing a refastenable article in accordance with one or more embodiments of the present disclosure.
FIG. 13 is a schematic example of a method for producing back side panels in accordance with one or more embodiments of the present disclosure.
FIG. 14a is a plan view showing the production of back side panels in accordance with one or more embodiments of the present disclosure.
FIG. 14b is a plan view showing the production of back side panels in accordance with one or more embodiments of the present disclosure.
FIG. 14c is a plan view showing the production of back side panels in accordance with one or more embodiments of the present disclosure.
FIG. 14d is a plan view showing the production of back side panels in accordance with one or more embodiments of the present disclosure.
FIG. 15 is a schematic representation of a method for manufacturing a refastenable article in accordance with one or more embodiments of the present disclosure.

### DETAILED DESCRIPTION

In the following description, numerous specific details are given to provide a thorough understanding of embodiments. The embodiments can be practiced without one or more of the specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The terms "first," "second," "third," and "fourth" as used herein in reference to side panels, breakable bonds, fastening components, edges and the like is not intended to refer to any specific order that the components are formed or added to a pant chassis during the manufacturing process or otherwise limit the claims to any specific embodiment illustrated or described herein. Instead, the terms are merely intended to clarify that a referenced component is different than a similar mentioned component.

### Refastenable Training Pants

Refastenable training pants are provided in a prefastened and folded configuration. The refastenable training pant includes a chassis having a leading edge, a trailing edge, and first and second lateral edges extending in a longitudinal direction between the leading edge and the trailing edge. The chassis is folded-over along a transverse fold line orthogonal to the first and second lateral edges. The refastenable training pant further includes a pair of first side panels attached to the chassis proximal one of the leading edge and the trailing edge of the chassis. The refastenable training pant also includes a pair of second side panels attached to the chassis proximal another of the leading edge and the trailing edge of the chassis. Each of the first and second side panels includes fastening components. The fastening components of the first and second side panels are refastenably engaged between the first and second lateral edges of the chassis. In some embodiments, the fastening components may comprise, for example, hook and loop fasteners.

The refastenable training pant also includes detachably secured first side panels. As used herein, the term "detachably secured" (or "detachably securing") means a temporary securement and/or attachment to the refastenable training pant, such that detaching the securement does not result in destruction of the components of the refastenable training pant or unfastening of the refastenable fastening components. In some embodiments, components may be detachably secured with a breakable bond between components. For example, the refastenable training pant may include a first breakable bond between each of the pair of first side panels and the chassis. In other embodiments, each of the pair of first side panels may be detachably secured in a respective pocket that is formed between a barrier leg cuff and the topsheet

The term "breakable bond" as used herein refers to a temporary connection made between components of the refastenable training pant that are weaker than the bonds that are employed to fix the side panels to the chassis such that when the refastenable article is opened and the breakable bonds are broken the side panels are not destroyed and the refastenable fastening components are not unfastened. The breakable bonds may be, for example, an adhesive bond, an ultrasonic bond, or a thermal bond.

In an exemplary embodiment, each of the first side panels may be folded inwardly into the chassis such that a first folded portion of each of the first side panels lies between the first and second lateral edges of the chassis. In such an embodiment, the refastenable training pant may include a first breakable bond between the first folded portion of each of the pair of first side panels and the chassis between the first and second lateral edges of the chassis. For example, each of the first breakable bonds may be located between the respective first side panel and the chassis. The first breakable bond may have a sufficiently weak bond strength such that the first breakable bond may be broken when the training pant is opened to be worn.

In an exemplary embodiment, each of the second side panels may be folded inwardly into the chassis such that a second folded portion of each of the second side panels lies between the first and second lateral edges of the chassis. In such an embodiment, the refastenable training pant may further include a second breakable bond between the second folded portion of each of the pair of second side panels and the chassis between the first and second lateral edges of the chassis. Each of the pair of second side panels may be folded outwardly over the second folded portion of each side panels such that a third folded portion of each of the second pair of side panels lies between the first and second lateral edges of the chassis. In such an embodiment, the refastenable training pant may further include a third breakable bond between the third folded portion of each of the second pair of side panels and the chassis between the first and second lateral edges of the chassis.

In some embodiments, the third breakable bond may be formed between at least a first tabbed portion of the third folded portion and the chassis, where the at least a first tabbed portion extends from a trailing edge or a leading edge of a distal region of the third folded portion. In still other embodiments, the refastenable training pant may further include a fourth breakable bond between the third folded portion of each of the second pair of side panels and the chassis between the first and second lateral edges of the chassis. In some embodiments, the fourth breakable bond may be formed between a second tabbed portion of the third folded portion and the chassis, where the second tabbed portion extends from another of the trailing edge or a leading edge of a distal region of the third folded portion.

In still other embodiments, the third breakable bond may be formed between the third folded portion and the second folded portion of each of the second side panels.

In some embodiments, each of the second side panels may be folded inwardly into the chassis such that a second folded portion of each of the second side panels lies between the first and second lateral edges of the chassis. Each of the pair of second side panels then may be folded outwardly over the second folded portion of each side panels such that a third folded portion of each of the second pair of side panels lies between the first and second lateral edges of the chassis. The second folded portion, in this embodiment, may not be bonded to the third folded portion. In such an embodiment, the refastenable training pant may further include a third breakable bond between the third folded portion of each of the second pair of side panels and the chassis between the first and second lateral edges of the chassis. In some embodiments, the third breakable bond may be formed between at least a first tabbed portion of the third folded portion and the chassis, where the at least a first tabbed portion extends from a trailing edge or a leading edge of a distal region of the third folded portion. In still other embodiments, the refastenable training pant may further include a fourth breakable bond between the third folded portion of each of the second pair of side panels and the chassis between the first and second lateral edges of the chassis. In some embodiments, the fourth breakable bond may be formed between a second tabbed portion of the third folded portion and the chassis, where the second tabbed portion extends from another of the trailing edge or a leading edge of a distal region of the third folded portion.

In other embodiments, the second folded portion may be bonded to the third folded portion. In still other embodiments, a third breakable bond may be formed between the third folded portion of each of the second side panels and the respective second folded portion of each of the second side panels between the first and second lateral edges of the chassis.

In still other embodiments, the refastenable training pant may not comprise a breakable bond. For example, each of the first side panels may be folded and then at least partially inserted into respective pockets located adjacent to each of the first and second lateral edges of the chassis. The respective pockets may detachably secure the folded portions of the first side panels to the chassis such that when the training pant is opened to be worn, the folded portions of the first side panels are released from the respective pockets.

In an exemplary embodiment, each of the first side panels may be folded inwardly over the chassis such that a first folded portion of each of the first side panels lies between the first and second lateral edges of the chassis. In such an embodiment, each of the first folded portions of the pair of first side panels may be folded and then at least partially inserted into respective pockets located adjacent to each of the first and second lateral edges of the chassis.

In an exemplary embodiment, each of the second side panels may be folded inwardly over the chassis such that a second folded portion of each of the second side panels lies between the first and second lateral edges of the chassis. Each of the pair of second side panels may be folded outwardly over the second folded portion of each of the side panels such that a third folded portion of each of the second pair of side panels lies between the first and second lateral edges of the chassis. In such an embodiment, each of the second folded portions and third folded portions of the pair of second side panels may be folded and then at least partially inserted into respective pockets located adjacent to each of the first and second lateral edges of the chassis.

In an exemplary embodiment, the refastenable training pant may include first side panels that include a fastening component comprising hook material. In some embodiments, the fastening component comprising hook material may be located on the non-body side of the first side panels. In some embodiments, the refastenable training pant may include second side panels that include a fastening component comprising loop material. In some embodiments, the fastening component comprising loop material may be located on the body side of the second side panels. In still other embodiments, the second side panels may comprise a loop material such that the second side panels may themselves serve as the fastening components.

As used herein, the term "non-body side" of a side panel means the portion of a side panel that is not facing towards the body of a person wearing the refastenable training pant, or, in other words, is facing outward from the body. As used herein, the term "body side" of a side panel means the portion of a side panel that faces towards the body of a person wearing the refastenable training pant, or, in other words, faces inwardly toward the body of the wearer.

The training pant chassis may comprise an at least partially fluid-permeable topsheet, a fluid-impermeable backsheet, and an absorbent core sandwiched between the topsheet and the backsheet. The topsheet and the backsheet may be bonded together along an outer perimeter. In such embodiments, each of the pair of first side panels may be permanently affixed to one of the first and second lateral edges of the chassis, and the first folded portions may be folded inwardly with respect to the outer perimeter over the topsheet so that the folded portion of each of the pair of first side panels may also be detachably secured to the chassis. In some embodiments, each folded portion of the pair of first side panels may be detachably secured to the chassis by a first breakable bond. In another embodiment, each folded portion of the pair of first side panels may be detachably secured to the chassis by at least partially inserting the folded portions into respective pockets.

In some embodiments, each of the pair of second side panels may be permanently affixed to one of the first and second lateral edges of the chassis, and each of the pair of second side panels is folded along a substantially S-shaped configuration and has second folded portions folded inwardly with respect to the outer perimeter of the chassis, over the topsheet along respective second fold lines and third folded portions folded along third fold lines over the respective second folded portions.

Each of the folded portions of the second side panels may also be detachably secured to the chassis. Each of the second folded portions may optionally be connected to the topsheet by a second breakable bond and each of the third folded portions may be connected to the chassis and/or topsheet by at least a third breakable bond. In still other embodiments, each of the third folded portions may instead be connected to the second folded portions by a third breakable bond. In yet other embodiments, each of the folded portions of the pair of second side panels may be detachably secured to the chassis by at least partially inserting the folded portions into respective pockets.

The first and/or second side panels may have respective lower edges shaped to anatomically conform to the legs of a wearer. In some embodiments, the first and second side panels may both be made of an elasticized material. In other embodiments, only one of the first and second side panels may be made of an elasticized material. Accordingly, in some embodiments, the first or second side panels may be made of a non-elasticized material. In some embodiments, one of the first and second fastening components may comprise a loop element formed by surface loops of the material constituting the corresponding side panel. In some embodiments, the first side panels may be substantially longer in a direction transverse to the first and second lateral edges than the second side panels. In another embodiment, the second side panels may be substantially longer in a direction transverse to the first and second lateral edges than the first side panels. In yet another embodiment, the first and second side panels may be substantially the same length in a direction transverse to the first and second lateral edges.

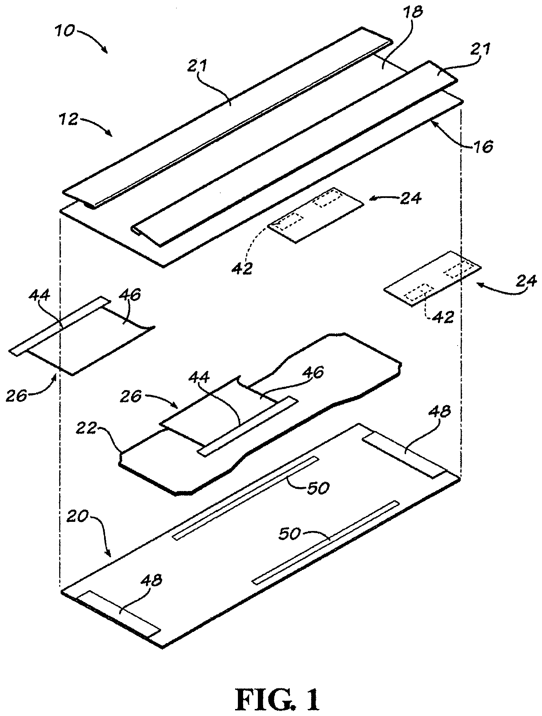

An exemplary embodiment of a refastenable training pant is illustrated in FIGs. 1-5. In FIGs. 1-5 the reference number **10** indicates a refastenable article **10** wearable as a pant-like garment. The refastenable article **10** comprises a chassis **12.** The chassis is defined by the two opposite lateral edges **14** and the front waist edge **41** and back waist edge **43.**

With reference to FIG. 1, the chassis **12** includes a topsheet **16** at least partially made of a fluid-permeable material, having an inner surface **18** which in use contacts the skin of the wearer.

The topsheet may be composed of a meltblown or spunbonded web of polyolefin fibers. The topsheet may also be a bonded-carded web composed of natural and/or synthetic fibers. The topsheet may be composed of a substantially hydrophobic material, and the hydrophobic material may, optionally, be treated with a surfactant or otherwise processed to impart a desired level of wettability and hydrophilicity. For example, the material may be surface treated with about 0.45 weight percent of a surfactant mixture comprising Ahcovel N-62 from Hodgson Textile Chemicals of Mount Holly, North Carolina U. S. A. and Glucopan 220UP from Henkel Corporation of Ambler, Pennsylvania in an active ratio of 3:1. The surfactant may be applied by any conventional means, such as spraying, printing, brush coating or the like. The surfactant may be applied to the entire topsheet **16** or may be selectively applied to particular sections of the topsheet, such as the medial section along the longitudinal center line.

A suitable liquid permeable topsheet **16** is a nonwoven bicomponent web having a basis weight of about 27 gsm (grams per square meter). The nonwoven bicomponent may be a spunbond bicomponent web, or a bonded carded bicomponent web. Suitable bicomponent staple fibers include a polyethylene/polypropylene bicomponent fiber available from CHISSO Corporation, Osaka, Japan. In this particular bicomponent fiber, the polypropylene forms the core and the polyethylene forms the sheath of the fiber.

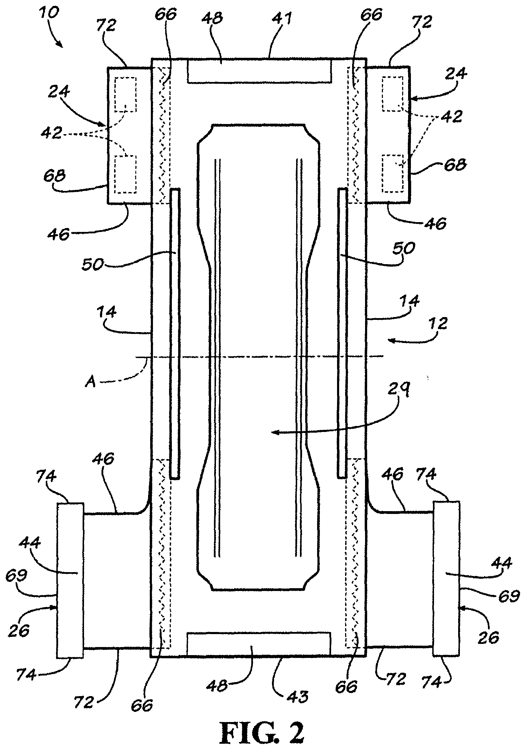

The topsheet **16** may include barrier leg cuffs **21** applied on the inner surface **18** which are configured to provide a barrier to the transverse flow of body exudates. A flap elastic member (FIG. 2) may be operatively joined with each leg cuff **21** in any suitable manner as is well known in the art. The leg cuffs **21** define a pair of edges, each assuming an upright configuration in at least the crotch region **29** of the refastenable article **10** to form a seal against the wearer's body. The elastic leg cuffs **21** may be located along the transversely opposed side edges, i.e., the lateral edges **14** of the chassis **12,** and may extend longitudinally along the entire length of the absorbent chassis or may only extend partially along the length of the chassis **12.** Suitable constructions and arrangements for the elastic leg cuffs **21** are generally well known to those skilled in the art and are described in U. S. Patent 4,704,116 issued November 3, 1987 to Enloe.

The chassis **12** may include a fluid-impermeable backsheet **20.** The backsheet **20** may be a single layer of liquid impermeable material, but desirably comprises a multilayered laminate structure in which at least one of the layers is liquid impermeable. For instance, the backsheet **20** may include a liquid permeable outer layer and a liquid impermeable inner layer that are suitably joined together by laminate adhesive, ultrasonic bonds, thermal bonds, or the like. Suitable laminate adhesives, which can be applied continuously or intermittently as beads, a spray, parallel swirls, or the like, can be obtained from Findley Adhesives, Inc., of Wauwatosa, Wisconsin U. S. A., or from National Starch and Chemical Company, Bridgwater, New Jersey U.S.A.

The liquid permeable outer layer can be any suitable material and desirably one that provides a generally cloth-like texture. One example of such a material is a 20 gsm (grams per square meter) spunbond polypropylene nonwoven web. The outer layer may also be made of those materials of which liquid permeable backsheet **20** is made. While it is not a necessity for outer layer to be liquid permeable, it is desired that it provides a relatively cloth-like texture to the wearer.

The inner layer of the backsheet **20** may be both liquid and vapor impermeable, or it may be liquid impermeable and vapor permeable. The inner layer may be manufactured from a thin plastic film, although other flexible liquid impermeable materials may also be used. The inner layer, or the liquid impermeable backsheet **20** when a single layer, prevents waste material from wetting articles, such as bedsheets and clothing, as well as the wearer and caregiver. A suitable liquid impermeable film for use as a liquid impermeable inner layer, or a single layer liquid impermeable backsheet **20,** is a 0.02 millimeter polyethylene film commercially available from Huntsman Packaging of Newport News, Virginia U. S. A. If the backsheet **20** is a single layer of material, it may be embossed and/or matte finished to provide a more cloth-like appearance. As earlier mentioned, the liquid impermeable material may permit vapors to escape from the interior of the disposable absorbent article, while still preventing liquids from passing through the backsheet **20.** A suitable "breathable" material may be composed of a microporous polymer film or a nonwoven fabric that has been coated or otherwise treated to impart a desired level of liquid impermeability. A suitable microporous film is a PMP-1 film material commercially available from Mitsui Toatsu Chemicals, Inc., Tokyo, Japan, or an XKO8044 polyolefin film commercially available from 3M Company, Minneapolis, Minnesota U. S. A.

The chassis **12** may also include an absorbent core **22.** The absorbent core **22** may be positioned between the backsheet **20** and the topsheet **16,** which components may be joined together by any suitable means such as adhesives, ultrasonic bonds, thermal bonds, or the like. The absorbent core **22** may be any structure which is generally compressible, conformable, non-irritating to the wearer's skin, and capable of absorbing and retaining liquids and certain body wastes. The absorbent core **22** may be manufactured in a wide variety of sizes and shapes, and from a wide variety of liquid absorbent materials commonly used in the art. For example, the absorbent core **22** may suitably comprise a matrix of hydrophilic fibers, such as a web of cellulosic fluff, mixed with particles of a high-absorbency material commonly known as superabsorbent material. In a particular embodiment, the absorbent core **22** may comprise a matrix of cellulosic fluff, such as wood pulp fluff, and superabsorbent hydrogel-forming particles. The wood pulp fluff may be exchanged with synthetic, polymeric, meltblown fibers or short cut homofil bicomponent synthetic fibers and natural fibers. The superabsorbent particles may be substantially homogeneously mixed with the hydrophilic fibers or can be nonuniformly mixed. The fluff and superabsorbent particles may also be selectively placed into desired zones of the absorbent core **22** to better contain and absorb body exudates. The concentration of the superabsorbent particles may also vary through the thickness of the absorbent core **22.** Alternatively, the absorbent core **22** may comprise a laminate of fibrous webs and superabsorbent material or other suitable means of maintaining a superabsorbent material in a localized area.

Suitable superabsorbent materials may be selected from natural, synthetic, and modified natural polymers and materials. The superabsorbent materials may be inorganic materials, such as silica gels, or organic compounds, such as crosslinked polymers, for example, sodium neutralized polyacrylic acid. Suitable superabsorbent materials are available from various commercial vendors, such as Dow Chemical Company located in Midland, Michigan U. S. A., and Stockhausen GmbH & Co. KG, D-47805 Krefeld, Federal Republic of Germany. Typically, a superabsorbent material is capable of absorbing at least about 15 times its weight in water, and desirably is capable of absorbing more than about 25 times its weight in water.

In one embodiment, the absorbent core **22** which can be rectangular or any other desired shape may comprise a blend of wood pulp fluff and superabsorbent material. One preferred type of pulp is identified with the trade designation CR1654, available from U. S. Alliance, Childersburg, Alabama U. S. A., and is a bleached, highly absorbent sulphate wood pulp containing primarily soft wood fibers and about 16 percent hardwood fibers. The superabsorbent material may be present in the absorbent core **22** in an amount of from 0 to about 90 weight percent based on total weight of the absorbent assembly. The absorbent core **22** suitably has a density within the range of about 0.10 to about 0.35 grams per cubic centimeter. The absorbent core **22** may or may not be wrapped or encompassed by a suitable tissue wrap that may help maintain the integrity and/or shape of the absorbent assembly.

The chassis **12** may also incorporate other materials that are designed primarily to receive, temporarily store, and/or transport liquid along the mutually facing surface with absorbent core **22,** thereby maximizing the absorbent capacity of the absorbent assembly. One suitable material is referred to as a surge layer (not shown) and comprises a material having a basis weight of about 50 to about 120 grams per square meter, and comprising a through-air-bonded-carded web of a homogenous blend of 60 percent 3 denier type T-256 bicomponent fiber comprising a polyester core/polyethylene sheath and 40 percent 6 denier type T-295 polyester fiber, both commercially available from Kosa Corporation of Salisbury, North Carolina U. S. A..

The topsheet **16** and the backsheet **20** may be bonded together along an outer perimeter of the chassis **12.**

In some embodiments, refastenable article **10** may be manufactured and sold with the chassis **12** folded-over along a central transversal fold line, indicated A in FIG. 2, transversal to the lateral edges **14** of the chassis.

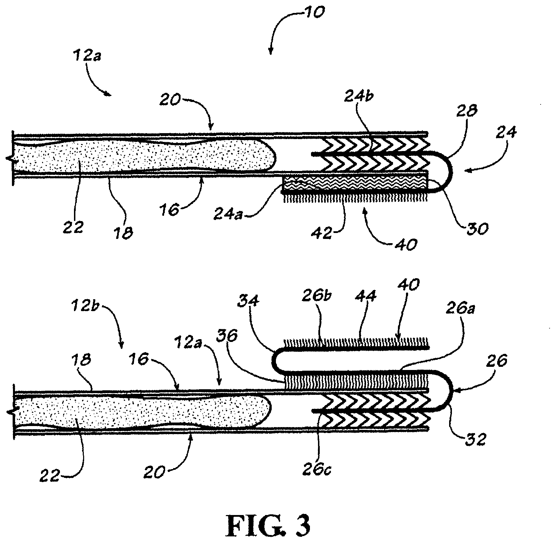

As shown in FIG. 3, in an exemplary configuration in which the refastenable article **10** may be manufactured and sold the chassis **12** has two folded portions **12a, 12b,** with respective portions of the inner surface **18** of the topsheet **16** facing each other.

The refastenable article **10** may comprise two front side panels **24** and two back side panels **26** fixed to respective lateral edges **14** of the chassis **12.** The terms "front" and "back" refer to the condition in which the refastenable article **10** is worn by the user. It should be noted that the orientation of the front side panels **24** and back side panels **26** may be reversed in some embodiments such that the back side panels **26** are worn in the front and the front side panels **24** are worn in the back.

These transversely opposed front side panels **24** and transversely opposed back side panels **26** can be permanently bonded along attachment lines **66** to the chassis **12** of the respective front and back waist regions. More particularly, as shown in FIGs. 2 and 3, the front side panels **24** may be permanently bonded to and extend transversely beyond the side edges **14** of the chassis **12** in the front waist region, and the back side panels **26** may be permanently bonded to and extend transversely beyond the side edges of the chassis **12** in the back waist region. The side panels **24** and **26** may be attached using attachment means known to those skilled in the art such as adhesive, thermal or ultrasonic bonding or the like. The front and back side panels **24** and **26** may be releasably attached to one another as illustrated by the fastening system **40.**

The illustrated side panels **24** and **26** each define a respective distal edge **68** and **69** that is spaced from the attachment line **66,** a leg end edge **46** disposed toward the longitudinal center of the training pant **10,** and a waist end edge **72** disposed toward a longitudinal end of the chassis. The leg end edge **46** and waist end edge **72** extend from the lateral edges **14** of the chassis **12** to the distal edges **68** and **69.** In the back waist region, the leg end edges **46** may be curved and/or angled relative to the transverse axis A to provide greater coverage toward the back of the pant as compared to the front of the pant. The waist end edges **72** may be parallel to the transverse axis A. The waist end edges **72** of the front side panels **24** form part of the front waist edge **41** of the absorbent refastenable article **10,** and the waist end edges **72** of the back side panels **26** form part of the back waist edge **43** of the refastenable article **10**.

Each of the side panels **24** and **26** may include one or more individual, distinct pieces of material. In particular embodiments, for example, each side panel **24** and **26** may include first and second side panel portions that are joined at a seam, or can include a single piece of material.

The side panels **24** and **26** may comprise an elastic material capable of stretching in a direction generally parallel to the transverse axis A of the training pant **10.** Suitable elastic materials are described in the following documents: EP 1 982 823 B1 and WO 2009/133508 A1.

With reference to FIG. 3, in the configuration in which the refastenable article **10** may be manufactured and sold the front side panels **24** have respective first folded portions **24a** folded along respective first fold lines **28** inwardly with respect to the outer perimeter of the chassis **12** over the inner surface **18** of the topsheet **16.** The first folded portions **24a** may be detachably secured to the inner surface **18** of the topsheet **16,** such as by respective first breakable bonds **30.** The front side panels **24** may have respective fixing portions **24b** which are fixed, e.g. by bonding, between the lateral edges of the topsheet **16** and backsheet **20.**

The back side panels **26** may be folded according to a substantially S-shaped configuration and may have respective second folded portions **26a** folded along respective second fold lines **32** inwardly with respect to the outer perimeter of the chassis **12** over the inner surface **18** of the topsheet **16.** The back side panels **26** may also have respective third folded portions **26b** folded along third fold lines **34** over the respective second folded portions **26a.** The back side panels **26** may have respective fixing portions **26c** which are fixed, e.g. by bonding, between the lateral edges of the topsheet **16** and backsheet **20.**

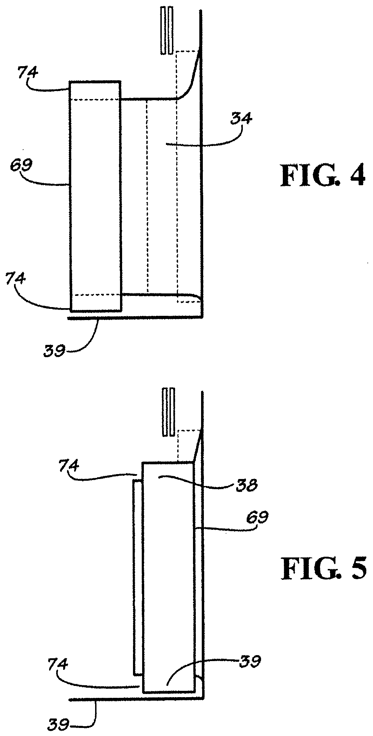

FIG. 4 shows details of the second breakable bond **36** that may be formed between the second folded portions **26a** and the topsheet **16.** As depicted, the second folded portions **26a** of the back side panels **26** may be folded inwardly with respect to the outer perimeter of the chassis **12** over the inner surface **18.** A second breakable bond **36** may then formed between the second folded portions **26a** and the topsheet **16.**

FIG. 5 shows details of the third breakable bond **38** and fourth breakable bond **39.** As depicted, the third folded portions **26b** of the back side panels **26** may be folded outwardly with respect to the outer perimeter of the chassis **12** over the inner surface **18.** A third and a fourth breakable bond **38, 39** may then be formed between the second folded portions **26b** and the topsheet **16.** The third and fourth breakable bonds **38, 39** may be formed between the first and second tabbed portions **74** that extend from the leading edge and trailing edge of a distal region of the back side panels **26** and the topsheet **16.**

In some embodiments, a second breakable bond **36** may not be formed. In such an embodiment, each of the pair of second side panels may be folded outwardly over the second folded portion **26a** of each side panels such that a third folded portion **26b** of each of the second pair of side panels lies between the first and second lateral edges of the chassis. In such an embodiment, the refastenable training pant may further include a third breakable bond **38** between the third folded portion **26b** of each of the second pair of side panels and the chassis between the first and second lateral edges of the chassis. In some embodiments, the third breakable bond **38** may be formed between at least a first tabbed portion **74** of the third folded portion and the chassis, where the at least a first tabbed portion **74** extends from a trailing edge or a leading edge of a distal region of the third folded portion **26b.** In still other embodiments, the refastenable training pant may further include a fourth breakable bond **39** between the third folded portion **26b** of each of the second pair of side panels and the chassis between the first and second lateral edges of the chassis. In some embodiments, the fourth breakable bond **39** may be formed between a second tabbed portion **74** of the third folded portion **26b** and the chassis, where the second tabbed portion **74** extends from another of the trailing edge or a leading edge of a distal region of the third folded portion **26b.**

In some embodiments, the third breakable bond **38** may be formed between a first tabbed portion **74** of the third folded portion **26b** of each of the second pair of side panels and the topsheet **16,** and the fourth breakable bond **39** may be formed between a second tabbed portion **74** of the third folded portion **26b** of each of the second pair of side panels and the topsheet **16.** In some embodiments, the first tabbed portion **74** extends from one of a trailing edge or a leading edge of a distal region of the third folded portion **26b** and the second tabbed portion **74** extends from another of the trailing edge and leading edge of the distal region of the third folded portion **26b.** The back side panels **26** have respective fixing portions **26c** which are fixed, e.g. by bonding, between the lateral edges of the topsheet **16** and backsheet **20.**

In still other embodiments, a third breakable bond **38** may be formed between the third folded portion **26b** of each of the second side panels and the respective second folded portion **26a** of each of the second side panels between the first and second lateral edges of the chassis.

The first folded portions **24a** of the front side panels **24** and the third folded portions **26b** of the back side panels **26** have respective mutually facing surfaces. The refastenable article **10** may include two fastening components that form refastenable closures **40.** Each fastening component may comprise a single fastening element or multiple fastening elements. The fastening components may comprise any refastenable fasteners suitable for absorbent articles, such as adhesive fasteners, cohesive fasteners, mechanical fasteners, or the like. In some embodiments, the side panel material itself may comprise the fastening component.

In particular embodiments the fastening components may comprise mechanical fastening elements for improved performance. Suitable mechanical fastening elements may be provided by interlocking geometric shaped materials, such as hooks, loops, bulbs, mushrooms, arrowheads, balls on stems, male and female mating components, buckles, snaps, or the like. In particular embodiments, the fastening components and mating fastening components comprise hook-and-loop fastening elements. One skilled in the art will recognize that the shape, density, and polymer composition of the hooks and loops may be selected to obtain the desired level of securement between the fastening components and the mating fastening components. A more aggressive hook material may comprise a material with a greater average hook height, a greater percentage of directionally-aligned hooks, or a more aggressive hook shape.

In one embodiment the refastenable article **10** may include a refastenable fastening system **40** comprising two refastenable closures, for example, complementary hook and loop fastening elements **42, 44** releasably engaged to each other. The hook and loop fastening elements **42, 44** may be fixed to the respective mutually facing surfaces of the first folded portions **24a** and third folded portions **26b.** The hook elements of the fastening system **40** may be provided either on the front side panels **24** or on the back side panels **26.** In one embodiment the loop elements of the fastening system **40** may be constituted by surface loops of the material constituting the corresponding side panels **24** or **26.**

It will be appreciated that, especially in FIG. 3, the relative proportions of parts and elements have been deliberately altered for ease of understanding. Also, in FIG. 3 the hook and loop fastening elements **42, 44** are shown separate (i.e. away) from each other. This is again done for ease of understanding, being otherwise understood that in the refastenable article **10** as finally produced, packaged, and sold, the hook and loop fastening elements **42, 44** of the fastening system **40** are engaged with each other to provide a closed condition of the refastenable article.

The first, second, third, and fourth breakable bonds **30, 36, 38, 39** may be formed by a layer of a so called "technical" or "green" glue or by a light bonding with thermal or ultrasonic bonding or the like. Suitable laminate adhesives, which may be applied continuously or intermittently as beads, a spray, parallel swirls, or the like, may be the AL11 obtained from Savarè I. C. of Milano, Italy. The temporary breakable bonds **30, 36, 38, 39** have the purpose of keeping the folded portions **24a, 26a, 26b** detachably secured to the respective inner surfaces **18** of the topsheet **16** in order to avoid that the bent portions **24a, 26a, 26b** undesirably "flap" outwardly with respect to the chassis **12** during the manufacturing process. The first, second, third, and fourth breakable bonds **30, 36, 38, 39** are designed to break when the refastenable article **10** is opened up to be worn. In some embodiments, the temporary breakable bonds **30, 36, 38, 39** may offer a peak resistance to breaking during the unfolding process of at least 25 N.

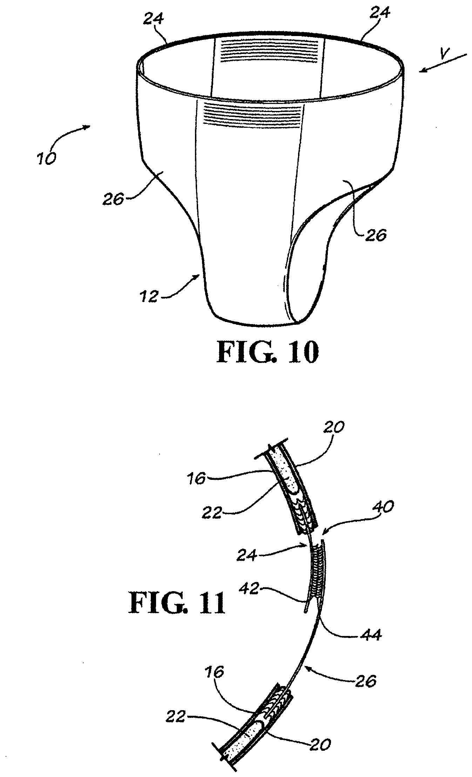

FIG. 10 shows the condition in which the refastenable article **10** may be opened so as to break the breakable bonds **30, 36, 38, 39.** In this condition the folded portions **24a, 26a, 26b,** after they are released from the detachable securement, may extend laterally outwardly from the chassis **12** at each of the opposite edges **14** to define respective portions of a waist line of the refastenable article **10.** The detail of FIG. 11 shows the connection between one front side panel **24** and the corresponding back side panel **26** after release from the temporary bonds.

Referring to FIGs. 1 and 2, in an embodiment the back side panels **26** may have respective leg end edges **46** shaped to anatomically conform to the legs of the wearer. In some embodiments, the back side panels **26** may have a first tabbed portion **74,** where the first tabbed portion **74** may be extending from one of a trailing edge and a leading edge of the distal region of the back side panels **26.** In some embodiments, the back side panels **26** may have two tabbed portions **74,** with a first tabbed portion **74** extending from one of a trailing edge and a leading edge of the distal region of the back side panel **26** and a second tabbed portion **74** extending from another of a trailing edge and a leading edge of the distal region of the back side panel **26.**

In an embodiment, either the front side panels **24** or the back side panels **26** may be made of elasticized material. Both the front side panels **24** and the back side panels **26** may be made of elasticized material.

In an embodiment the refastenable article **10** may include two elastic waist members **48** extending on two opposite waist portions of the chassis **12** respectively between the two front side panels **24** and between the two back side panels **26.** In other embodiments, the two elastic waist members **48** may not extend the entire distance between the two front side panels **24** and between the two back side panels **26.** In still other embodiments, the refastenable article **10** may include two or more elastic waist members **48** extending between the two front side panels **24** and between the two back side panels **26.**

In an embodiment, the chassis **12** may further include two elastic leg members **50** extending parallel to the opposite lateral edges **14** to provide a close fit of the refastenable article **10** around the wearer's legs. In other embodiments, the chassis **12** may include two or more elastic leg members **50** to provide a close fit of refastenable article **10** around the wearer's legs.

The elastic waist members **48** and the elastic leg members **50** may be formed of any suitable elastic material. As is well known to those skilled in the art, suitable elastic materials include sheets, strands or ribbons of natural rubber, synthetic rubber, or thermoplastic elastomeric polymers. The elastic materials may be stretched and adhered to a substrate, adhered to a gathered substrate, or adhered to a substrate and then elasticized or shrunk, for example with the application of heat; such that elastic constrictive forces are imparted to the substrate. In one particular embodiment, for example, the elastic leg members **50** comprise a plurality of dry-spun coalesced multifilament spandex elastomeric threads sold under the trade name LYCRA and available from INVISTA, INVISTA Building, 4123 East 37th Street North, Wichita, KS 67220 U. S. A.

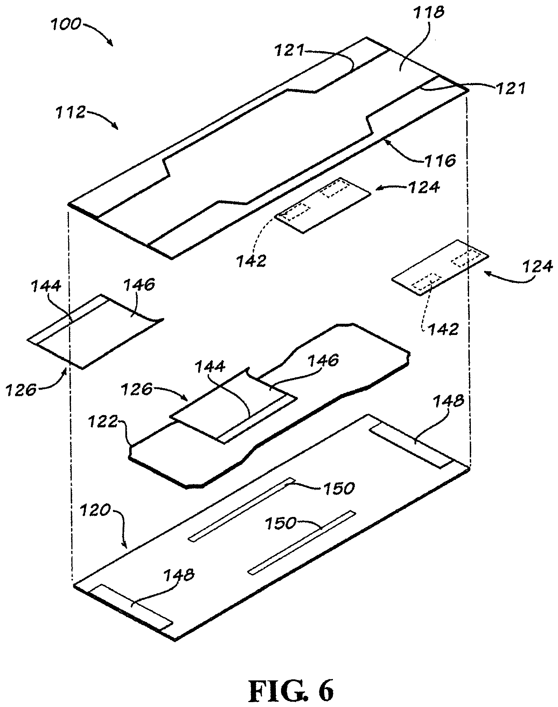

Another exemplary embodiment of a refastenable training pant is illustrated in FIGs. 6-9. In FIGs. 6-9 the reference number **100** indicates a refastenable article **100** wearable as a pant-like garment. The refastenable article **100** comprises a chassis **112**. The chassis **112** is defined by the two opposite lateral edges **114** and the front waist edge **141** and back waist edge **143.**

With reference to FIG. 6, the chassis **112** may include a topsheet **116** at least partially made of a fluid-permeable material, having an inner surface **118** which in use contacts the skin of the wearer.

The topsheet may be composed of a meltblown or spunbonded web of polyolefin fibers. The topsheet may also be a bonded-carded web composed of natural and/or synthetic fibers. The topsheet may be composed of a substantially hydrophobic material, and the hydrophobic material may, optionally, be treated with a surfactant or otherwise processed to impart a desired level of wettability and hydrophilicity. The surfactant may be applied to the entire topsheet **116** or may be selectively applied to particular sections of the topsheet, such as the medial section along the longitudinal center line.

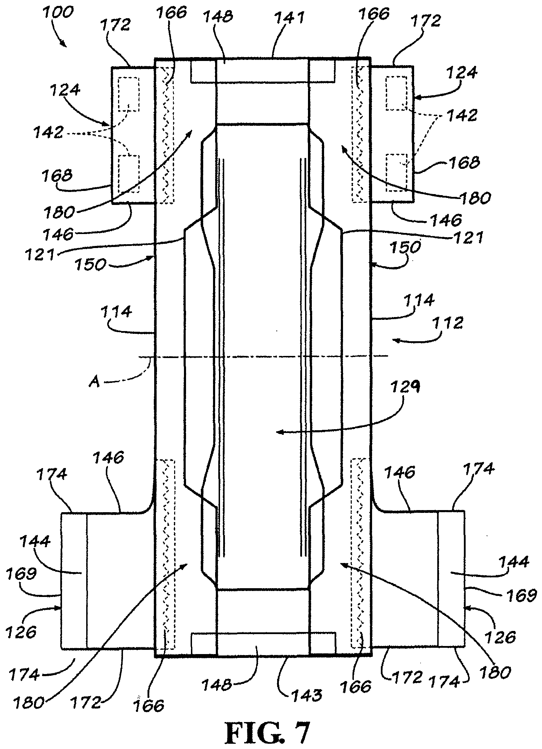

The topsheet **116** may include barrier leg cuffs **121** applied on the inner surface **118** which are configured to provide a barrier to the transverse flow of body exudates. The leg cuffs **121** define a pair of edge regions, each assuming an upright configuration in at least the crotch region **129** of the refastenable article **110** to form a seal against the wearer's body. The elastic leg cuffs **121** may be located along the transversely opposed side edges, i.e., the lateral edges **114** of the chassis **112,** and may extend along the length of the chassis **112.** As illustrated in FIG. 7, the leg cuffs **121** may be configured to provide four pockets **180** at the corners of the chassis **112.** The pockets **180** may be formed between the inner surface **118** of the topsheet **116** and the leg cuffs **121.**

The chassis **112** may include a fluid-impermeable backsheet **120.** The backsheet **120** may be a single layer of liquid impermeable material, but may comprise a multilayered laminate structure in which at least one of the layers may be liquid impermeable.

The inner layer of the backsheet **120** may be both liquid and vapor impermeable, or it may be liquid impermeable and vapor permeable. The inner layer may be manufactured from a thin plastic film, although other flexible liquid impermeable materials may also be used. The inner layer, or the liquid impermeable backsheet **120** when a single layer, prevents waste material from wetting articles, such as bedsheets and clothing, as well as the wearer and caregiver.

The chassis **112** may also include an absorbent core **122.** The absorbent core **122** may be positioned between the backsheet **120** and the topsheet **116,** which components may be joined together by any suitable means such as adhesives, ultrasonic bonds, thermal bonds, or the like. The absorbent core **122** may be any structure which is generally compressible, conformable, non-irritating to the wearer's skin, and capable of absorbing and retaining liquids and certain body wastes. The absorbent core **122** may be manufactured in a wide variety of sizes and shapes, and from a wide variety of liquid absorbent materials commonly used in the art.

The chassis **112** may also incorporate other materials that are designed primarily to receive, temporarily store, and/or transport liquid along the mutually facing surface with absorbent core **122,** thereby maximizing the absorbent capacity of the absorbent assembly.

The topsheet **116** and the backsheet **120** may be bonded together along an outer perimeter of the chassis **112.**

The transversely opposed front side panels **124** and transversely opposed back side panels **126** may be permanently bonded along attachment lines **166** to the chassis **112** of the respective front and back waist regions. More particularly, as shown in FIGs. 6 and 7, the front side panels **124** may be permanently bonded to and extend transversely beyond the side edges **114** of the chassis **112** in the front waist region, and the back side panels **126** may be permanently bonded to and extend transversely beyond the side edges of the chassis **112** in the back waist region. The side panels **124** and **126** may be attached using attachment means known to those skilled in the art such as adhesive, thermal or ultrasonic bonding or the like. The front and back side panels **124** and **126** may be releasably attached to one another as illustrated by the fastening system **140.**

The illustrated side panels **124** and **126** each define a respective distal edge **168** and **169** that is spaced from the attachment line **166,** a leg end edge **146** disposed toward the longitudinal center of the training pant **100,** and a waist end edge **172** disposed toward a longitudinal end of the chassis. The leg end edge **146** and waist end edge **172** extend from the lateral edges **114** of the chassis **112** to the distal edges **168** and **169.** In the back waist region, the leg end edges **146** may be curved and/or angled relative to the transverse axis A to provide greater coverage toward the back of the pant as compared to the front of the pant. The waist end edges **172** may be parallel to the transverse axis A. The waist end edges **172** of the front side panels **124** form part of the front waist edge **141** of the refastenable article **100,** and the waist end edges **172** of the back side panels **126** form part of the back waist edge **143** of the refastenable article **100.**

Each of the side panels **124** and **126** may include one or more individual, distinct pieces of material. In particular embodiments, for example, each side panel **124** and **126** may include first and second side panel portions that are joined at a seam, or can include a single piece of material.

The side panels **124** and **126** may comprise an elastic material capable of stretching in a direction generally parallel to the transverse axis A of the training pant **100.** In some embodiments, the side panels **124** and **126** may all comprise an elastic material. In other embodiments, only side panels **124** may comprise an elastic material. In still other embodiments, only side panels **126** may comprise an elastic material.

In embodiments, the refastenable article **100** may be manufactured and sold with the chassis **112** folded-over along a central transversal fold line, indicated A in FIG. 7, transversal to the lateral edges **114.**

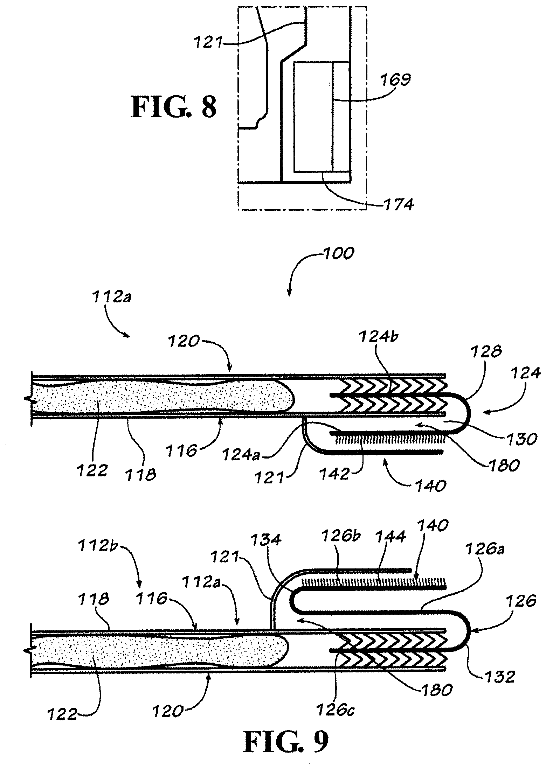

As shown in FIG. 9, in an exemplary configuration in which the refastenable article **100** may be manufactured and sold the chassis **112** has two folded portions **112a, 112b,** with respective portions of the inner surface **118** of the topsheet **116** facing each other.

The refastenable article **100** may comprise two front side panels **124** and two back side panels **126** fixed to respective lateral edges **114** of the chassis **112.** These transversely opposed front side panels **124** and transversely opposed back side panels **126** may be permanently bonded along attachment lines **166** to the chassis **112** of the respective front and back waist regions. More particularly, as shown in FIG. 7, the front side panels **124** may be permanently bonded to and extend transversely beyond the side edges **114** of the chassis **112** in the front waist region, and the back side panels **126** may be permanently bonded to and extend transversely beyond the side edges of the chassis **112** in the back waist region. The front side panels **124** may have respective fixing portions **124b** which are fixed, e.g. by bonding, between the lateral edges of the topsheet **116** and backsheet **120.** The side panels **124** and **126** may be attached using attachment means known to those skilled in the art such as adhesive, thermal or ultrasonic bonding or the like. The front and back side panels **124** and **126** may be releasably attached to one another as illustrated by the fastening system **140.**

With reference to FIG. 9, in the configuration in which the refastenable article **100** may be manufactured and sold the front side panels **124** may have respective first folded portions **124a** folded along respective first fold lines **128** inwardly with respect to the outer perimeter of the chassis **112** over the inner surface **118** of the topsheet **116.** The first folded portions **124a** may be detachably secured to the inner surface **118** of the topsheet **116** by inserting at least a portion of the first folded portions **124a** of the front side panels **124** into a pocket **180** formed between the inner surface **118** of the topsheet **116** and leg cuffs **121.**

The back side panels **126** may be folded according to a substantially S-shaped configuration and have respective second folded portions **126a** folded along respective second fold lines **132** inwardly with respect to the outer perimeter of the chassis **112** over the inner surface **118** of the topsheet **116.** The back side panels **126** may also have respective third folded portions **126b** folded along third fold lines **134** over the respective second folded portions **126a.** The back side panels **126** have respective fixing portions **126c** which are fixed, e.g. by bonding, between the lateral edges of the topsheet **116** and backsheet **120.**

FIG. 8 shows details of the folding of the back side panels **126.** As depicted, the second folded portions **126a** of the back side panels **126** may be folded inwardly with respect to the outer perimeter of the chassis **112** over the inner surface **118.** As depicted, the third folded portions **126b** of the back side panels **126** may be folded outwardly with respect to the outer perimeter of the chassis **112** over the inner surface **118.** As illustrated in FIG. 8, leg cuffs **121** may be configured to accommodate the folded back side panels **126** such that the folded back side panels **126** may be detachably secured in a pocket **180** that is formed between the inner surface **118** of the topsheet **116** and the leg cuffs **121.**

The first folded portions **124a** of the front side panels **124** and the third folded portions **126b** of the back side panels **126** may have respective mutually facing surfaces. The refastenable article **100** may include two fastening components that form refastenable closures **140.** Each fastening component may comprise a single fastening element or multiple fastening elements. The fastening components may comprise any refastenable fasteners suitable for absorbent articles, such as adhesive fasteners, cohesive fasteners, mechanical fasteners, or the like. In some embodiments, the side panel material itself may comprise the fastening component.

The refastenable article **100** may be opened so as to release the side panels **124** and **126** from their respective pockets **180.** In this condition the folded portions **124a, 126a,** and **126b,** after they are released from the detachable securement, extend laterally outwardly from the chassis **12** at each of the opposite edges **114** to define respective portions of a waist line of the refastenable article **110.** The detail of FIG. 10 shows refastenable article **10,** which is analogous to refastenable article **100** for this illustration, that opens into a similar training pant product. The detail of FIG. 11, showing refastenable article **10,** also by analogy, shows the details of refastenable article **100,** with the connection between one front side panel **124** and the corresponding back side panel **126** after release from the respective pockets **180.**

### Methods of Manufacturing_Refastenable Training Pants

Methods of preparing a refastenable article are also disclosed. In some embodiments, the methods include providing a refastenable training pant comprising a chassis having a leading edge, a trailing edge, and a first and a second lateral edge extending in a longitudinal direction between the leading edge and the trailing edge. The refastenable training pant may include a first pair of side panels attached to the chassis proximal one of the leading edge and the trailing edge of the chassis and a second pair of side panels attached to the chassis proximal another of the leading edge and the trailing edge of the chassis. The first pair of side panels each comprises a first fastening component and the second pair of side panels each comprises a second fastening component. The first pair of side panels is folded inwardly over the chassis such that the first fastening components lie between the first and second lateral edges of the chassis, and the second pair of side panels is folded inwardly over the chassis such that the second fastening components lie between the first and second lateral edges of the chassis. The method further includes refastenably engaging the first fastening components and second fastening components while the first fastening components and second fastening components lie between the first and second lateral edges of the chassis.

In some embodiments, the method further includes transporting the refastenable training pant in a machine direction to a folding device. The refastenable training pant may be transported to the folding device with the first pair of side panels are folded inwardly over the chassis such that the first fastening components lie between the first and second lateral edges of the chassis, and the second pair of side panels are folded inwardly over the chassis such that the second fastening components lie between the first and second lateral edges of the chassis. In certain embodiments, the method further includes folding the refastenable training pant about a transverse fold line with the folding device such that the first fastening components of the first pair of side panels face the second fastening components of the second pair of side panels between the first and second lateral edges of the chassis.

In some embodiments, the method may further include folding each of the first pair of side panels inwardly over the chassis such that a first folded portion of each of the first side panels and the first fastening components lie between the first and second lateral edges of the chassis. In some embodiments, the method may include detachably securing the first folded portion of each of the first pair of side panels to the chassis between the first and second lateral edges of the chassis. In certain embodiments, the method may include forming a first breakable bond between the first folded portion of each of the first pair of side panels and the chassis between the first and second lateral edges of the chassis. In other embodiments, the method may include detachably securing the first folded portions to the chassis using respective pockets **180** formed between the chassis **112** and the leg cuffs **121.**

In certain embodiments, the method may further include folding each of the second pair of side panels inwardly over the chassis such that a second folded portion of each of the second side panels lies between the first and second lateral edges of the chassis. In such embodiments, a second breakable bond may be formed between the second folded portion of each of the second pair of side panels and the chassis between the first and second lateral edges of the chassis. In certain embodiments, the method may further include folding each of the second pair of side panels outwardly over the second folded portion of each side panels such that a third folded portion of each of the second pair of side panels lies between the first and second lateral edges of the chassis. In such embodiments, at least a third breakable bond may be formed between the third folded portion of each of the second pair of side panels and the chassis between the first and second lateral edges of the chassis. In some embodiments, a fourth breakable bond may be formed between the third folded portion of each of the second pair of side panels and the chassis between the first and second lateral edges of the chassis.

In still other embodiments, a third breakable bond may be formed between the third folded portion of each of the second side panels and the respective second folded portion of each of the second side panels between the first and second lateral edges of the chassis.

In some embodiments, the method may include detachably securing the second and third folded portions of each of the second pair of side panels to the chassis between the first and second lateral edges of the chassis. In embodiments, the method may include detachably securing the second and third folded portions to the chassis using respective pockets **180** formed between the topsheet **116** and the leg cuffs **121.**

In some embodiments, the refastenable training pant may be formed by a method which includes: providing a topsheet for forming a portion of a pant chassis; applying a pair of first side panels to the topsheet, each of the pair of first side panels having a refastenable fastening element; folding each of the first side panels to form first folded portions (for example, by folding the first folded portions over the topsheet); and detachably securing the first folded portion of each of the first side panels to the pant chassis. As used herein, the terms "chassis" and "pant chassis" are equivalent.

In an exemplary embodiment, the topsheet may be provided in a form of a continuous web, and the first side panels may be applied to the topsheet, folded, and the first breakable bonds may be formed while the topsheet is in the form of the continuous web. The topsheet has a first and a second lateral edge and the first breakable bonds may be formed between the first and second lateral edges of the topsheet.

In another exemplary embodiment, the topsheet may be provided in the form of a continuous web, the first side panels may be applied to the topsheet, folded, and at least partially inserted into respective pockets **180** formed between the chassis and the leg cuffs **121.**

In some embodiments, the method of forming the refastenable training pant may further include affixing a pair of second side panels to the topsheet. The second side panels may be folded to form third folded portions. The second side panels may be folded over the topsheet to form second folded portions. Second breakable bonds may be formed between the second folded portions and the topsheet. Third and fourth breakable bonds may be formed between the third folded portions and the topsheet. In some embodiments, a third breakable bond may instead be formed between the third folded portions and the second folded portions.

In other embodiments, the method of forming the refastenable training pant may further include affixing a pair of second side panels to the topsheet. The second side panels may be folded to form third folded portions. The second side panels may be folded over the topsheet to form second folded portions. The second and third folded portions of the second side panels may each be at least partially inserted into respective pockets **180** formed between the chassis and the leg cuffs **121.**

In some embodiments, the method may further include providing a continuous web material forming a backsheet which may be superposed and fixed to the topsheet and the first and second side panels.

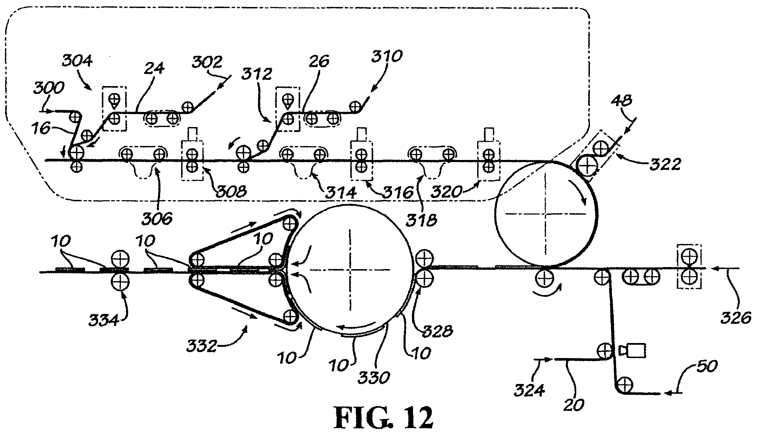

FIG. 12 is a schematic example of a process adapted for producing training pants as exemplified in FIGs. 1-5. In this example, the first side panels may be attached to the chassis before the second side panels are attached to the chassis. It should be noted, however, that the first side panels may be attached to the chassis either before or after the second side panels are attached to the chassis.

In the representation of FIG. 12, reference number **300** denotes the source of a continuous web material forming the topsheet **16.** The reference number **302** indicates the source of continuous web materials forming the front side panels **24.** The front side panels **24** may include the respective fastening elements **44** of the refastenable fastening system **40.** In a first side panel attachment device **304,** the front side panels **24** may be cut and then applied on the topsheet **16.** For example, the front side panels **24** may be bonded to the topsheet **16** with thermal or ultrasonic bonding or the like.

In a first side panel folding device **306,** the front side panels **24** may be folded over the topsheet **16** to form the first folded portions **24a.** As used herein, the terms "folding unit," "side panel folding device," and "folding device" are equivalent. In a first securing device **308,** the first breakable bonds **30** may be formed. In some embodiments, the first securing device **308** may be a breakable bond forming device. In embodiments in which the breakable connect is formed by thermal or ultrasonic bonding, a thermal or ultrasonic bonding device may be employed to form a thermal or ultrasonic bond to form the first breakable bonds **30.** Alternatively, the breakable connect may be formed with an adhesive. When an adhesive is used, the adhesive may be applied before folding the front side panels **24.**

The reference number **310** indicates the source of at least one continuous web material forming the back side panels **26.** The back side panels **26** may include the respective fastening elements **42** of the refastenable fastening system **40.** In a second side panel attachment device **312** the back side panels **26** may be cut and then applied on the topsheet **16.** In a second side panel folding device **314** the back side panels **26** may be folded over topsheet **16** to form the second folded portions **26a.** In a second securing device **316** the second breakable bonds **36** may be formed. In embodiments in which the breakable connect is formed by thermal or ultrasonic bonding, a thermal or ultrasonic bonding device may be employed to form a thermal or ultrasonic bond to form the second breakable bonds **36.** Alternatively, the breakable connect may be formed with an adhesive. When an adhesive is used, the adhesive may be applied before folding the back side panels **26.**

In a third side panel folding device **318,** the back side panels may be folded over along a third folding line to form the third folded portions **26b.** In a third securing device **320,** the third and fourth breakable bonds **38, 39** may be formed. In embodiments in which the breakable connect is formed by thermal or ultrasonic bonding, a thermal or ultrasonic bonding device may be employed to form a thermal or ultrasonic bond to form the third and fourth breakable bonds **38, 39.** Alternatively, the breakable connect may be formed with an adhesive. When an adhesive is used, the adhesive may be applied before folding the back side panels **26.** In still other embodiments, the third securing device **320** may be used to make a third breakable bond between the third folded portion of each of the second side panels and the respective second folded portion of each of the second side panels between the first and second lateral edges of the chassis.

An elastic web band attachment unit **322** may apply elastic waist bands **48** to the topsheet **16.**

The reference number **324** denotes the source of a continuous web material forming the backsheet **20.** Elastic leg members **50** may be applied on the backsheet **20.** Absorbent cores **22** coming from a source **326** may then applied to the backsheet **20.**

The topsheet **16** with the front and back side panels **24, 26** and elastic waist bands **48** may then be superposed and fixed to the backsheet **20** with the elastic leg members **50** and absorbent cores **22.**

A cutting station **328** cuts transversally the continuous webs to form blanks of refastenable articles **10** which are maintained by vacuum on the outer surface of a roller **330.**

A folder **332** folds the refastenable articles **10** along respective transverse folding line A. In some embodiments, the folder **332** may maintain the refastenable articles **10** in position during folding by vacuum. For example, the folder **332** may include vacuum nip rolls and/or vacuum conveyors. In other embodiments, the folder **332** may fold the refastenable articles **10** without the assistance of vacuum. Advantageously, it has been found that commercial production speeds may be achieved without the use of vacuum because of the stability provided by the breakable bonds to the side panels and the placement of the folded portions **24a, 26a,** and **26b** of the side panels **24** and **26** inwardly with respect to the outer perimeter of the chassis **12.**

When folding the refastenable article **10** in the folder **332,** the refastening elements **42, 44** of the refastenable fastening system **40** and the front and back side panels **24, 26** may be maintained within the chassis between the folded portions **24a, 26a,** and **26b** and the lateral edges **14** by the breakable bonds.

In some embodiments, the transversal folding operation performed in the folder **332** may bring the hook and loop fastening elements **42, 44** of the refastenable fastening system **40** into contact with each other. In other embodiments, the transversal folding operation performed by the folder **332** may be performed while maintaining separation between the hook and loop fastening elements **42, 44,** e.g., with the assistance of vacuum. The hook and loop elements may be connected to each other by pressure rollers **334** to form the final refastenable articles **10.** The refastenable articles **10** may thus be closed to the "pre-fastened" condition in which the refastenable article **10** may be packaged and sold.

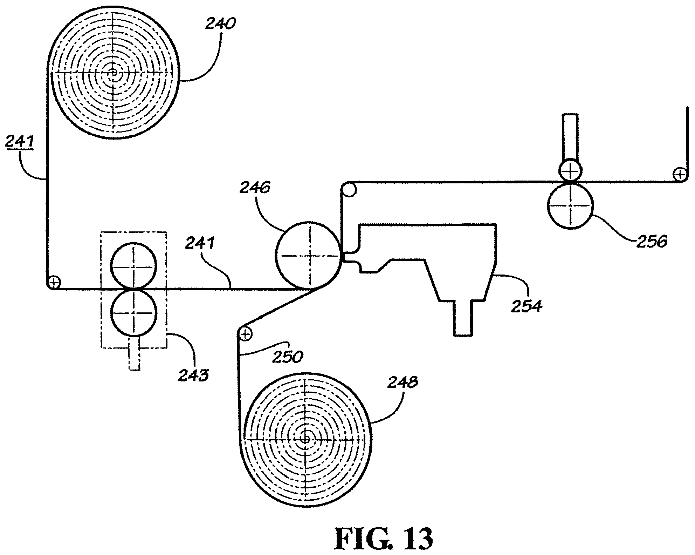

In some embodiments, a method for making back side panels **26** is provided. FIG. 13 is a schematic example of a method for producing back side panels **26.**

In the representation of FIG. 13, reference number **240** denotes the source of a continuous web material **241** forming the back side panels **26.** The side panel continuous web material **241** may extend between two longitudinal side edges **262** and has a length substantially parallel to the longitudinal edges. At a cutting unit **243,** at least a portion **245** of the web material **241** forming the back side panels **26** may be cut from the continuous web at a repeating interval. In some embodiments, the repeating interval may be determined based on the desired size of the back side panels **26.** In some embodiments, the at least a portion **245** of the web material **241** may be cut from a medial region between the two side edges **262.** As used herein, the term "medial region" means an area that is centrally located between the two side edges **262.**

The reference number **248** indicates the source of a continuous web material forming a loop material **250.** The loop material **250** may be applied along the length of the back side panel continuous web material **241** between the longitudinal edges **262** of the back side panel continuous web material in the medial region between the two longitudinal edges. A joining roller **246** may be used to combine the side panel continuous web material **241** with the continuous web material of the loop material **250** and the web materials may be bonded together using a bonding unit **254.** Bonding may be accomplished by any means known to those of ordinary skill in the art, including but not limited to ultrasonic bonding. Finally, the combined web materials may pass through a slitter unit **256** that cuts the combined web materials along the length of the side panel continuous web material **241** near the center of the combined side panel continuous web material **241** and continuous web of the loop material **250** between the two side edges **262.** In some embodiments, the combined web materials may further be cut into individual pairs of back side panels **26** by cutting across the combined side panel continuous web material **241** and continuous web of the loop material **250** transversely to the length of the side panel continuous web material **241** in the region where the at least a portion **245** of web material forming the back side panels has been cut from the continuous web.

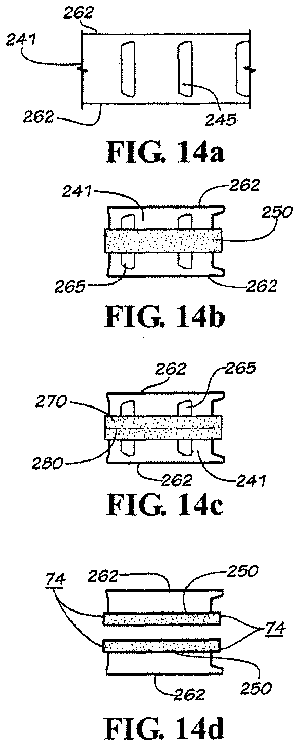

As illustrated in FIG. 14a, at least a portion of the web material forming the back side panels may be cut from the continuous web at a repeating interval. In some embodiments, the cut portion **245** may be in the shape of a trapezoid. In other embodiments, the cut portion **245** may be in the shape of a rectangle or square.

In the representation of FIG. 14b, the band of loop material **250** is shown as may be applied to the web material **241** forming the back side panels **26.** In the embodiment illustrated in FIG.14b, the band of loop material **250** may be applied between the two side edges **262.** In some embodiments, the band of loop material **250** may be applied medially between the two side edges **262.**

In the representation of FIG. 14c, and in some embodiments, the combined web materials are shown as being cut at the center between of the two side edges **262.** In some embodiments, the combined web materials may further be cut into individual pairs of back side panels **26** by separating in the region where the at least a portion of web material forming the back side panels, the cut portion **245,** has been cut from the back side panel continuous web material **241.**

As shown in FIG. 14d, the cut back side panels **26** may include the tabbed portions **74** described hereinabove. In some embodiments, these tabbed portions may be formed by the continuous web of the loop material **250.**

In some embodiments, the sequence of steps illustrated by Figures 13 and 14a-14d may be performed in an offline process. In other embodiments, the combined side panel continuous web material **241** and continuous web of the loop material **250** illustrated in FIG. 14c may be slit longitudinally with the slitter unit **256** as described above and then fed into the refastenable article production process as the source of continuous web materials **102** forming the back side panels **26** that may be individually cut and then attached to the topsheet **16.** This training pant production process is illustrated in FIG. 12.

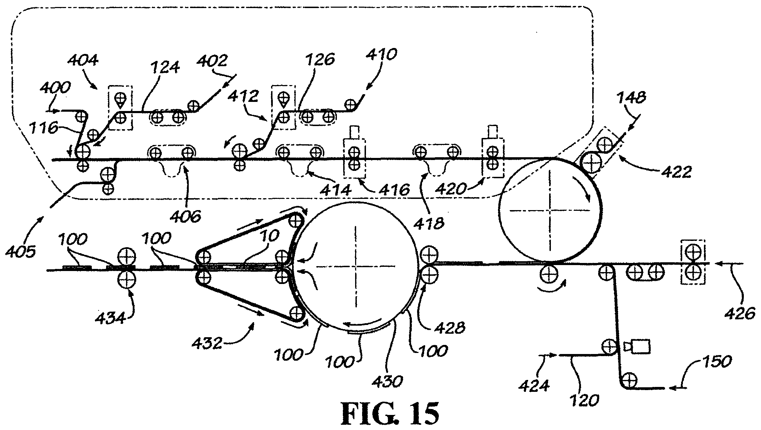

FIG. 15 is a schematic example of a process adapted for producing training pants as exemplified in FIGs. 6-9.

In the representation of FIG. 15, reference number **400** denotes the source of a continuous web material forming the topsheet **116.** The reference number **402** indicates the source of continuous web materials forming the front side panels **124.** The front side panels **124** may include the respective fastening elements **144** of the refastenable fastening system **140.** In a first side panel attachment device **404,** the front side panels **124** may be cut and then may be applied on the topsheet **116.** For example, the front side panels **124** may be bonded to the topsheet **116** with thermal or ultrasonic bonding or the like.

A leg cuff attachment unit **405** may be provided for attaching the leg cuffs **121** to the topsheet **116.** The leg cuff attachment unit **405** may attach the leg cuffs **121** to the topsheet **116** with an adhesive or by thermal or ultrasonic bonding. The leg cuffs **121** may be configured to create pockets **180** adjacent to the corners of the topsheet **116.** The pockets **180** may be used to at least partially secure the folded front and back side panels **124, 126** such that the transversal folding operation performed in the folder **432** will bring the hook and loop fastening elements **142, 144** of the refastenable fastening system **140** into contact with each other.

In a first side panel folding device **406,** the front side panels **124** may be folded over the topsheet **116** to form the first folded portions **124a.** In a first securing device **416,** the folded front side panels **124** may be at least partially inserted (i.e., tucked) into respective pockets **180** that may be located adjacent to one of the first and second lateral edges of the chassis. In some embodiments, the first securing device **416** may comprise a tucking device. In some embodiments, the first securing device **416** may at least partially insert the folded front side panels **124** into respective pockets **180** that are formed between the leg cuffs **121** and the topsheet **118** of the chassis **116.**

The reference number **410** indicates the source of at least one continuous web material forming the back side panels **126.** The back side panels **126** may include the respective fastening elements **142** of the refastenable fastening system **140.** In a second side panel attachment device **412** the back side panels **126** may be cut and then applied on the topsheet **116.** In a second side panel folding device **414** the back side panels **126** may be folded over topsheet **116** to form the second folded portions **126a.** In a third side panel folding device **418** the back side panels **126** may again be folded over to form the second folded portions **126b.**

In a second securing device **420** the folded back side panels **126** may be at least partially inserted (i.e., tucked) into respective pockets **180** that may be located adjacent to one of the first and second lateral edges of the chassis **116.** In some embodiments, the second securing device **420** may at least partially insert the folded back side panels **126** into respective pockets **180** that may be formed between the leg cuffs **121** and the topsheet **118** of the chassis **116.**

An elastic web band attachment unit **422** may apply elastic web bands **148** to the topsheet **116.**

The reference number **424** denotes the source of a continuous web material forming the backsheet **120.** Elastic leg members **150** may be applied on the backsheet **120.** Absorbent cores **122** coming from a source **426** may then be applied to the backsheet **120.**

The topsheet **116** with the front and back side panels **124, 126** and elastic waist band **148** may then be superposed and fixed to the backsheet **120** with the elastic leg members **150** and absorbent cores **122.**

A cutting station **428** cuts transversally the continuous webs to form blanks of refastenable articles **100** which may be maintained by vacuum on the outer surface of a roller **430.**

A folder **432** may fold the refastenable articles **100** along respective transverse folding line A. In some embodiments, the folder **432** may maintain the refastenable articles **100** in position during folding by vacuum. For example, the folder **432** may include vacuum nip rolls and/or vacuum conveyors. In other embodiments, the folder **432** may fold the refastenable articles **100** without the assistance of vacuum.

When folding the refastenable article **100** in the folder **432,** the refastening elements **142, 144** of the refastenable fastening system **140** and the front and back side panels **124, 126** may be maintained between the folded portions **124a, 126a,** and **126b** and the lateral edges **114** by the means of detachable securement, for instance a pocket **180.**

In some embodiments, the transversal folding operation performed in the folder **432** may bring the hook and loop fastening elements **142, 144** of the refastenable fastening system **140** into contact with each other. In other embodiments, the transversal folding operation performed by the folder **432** may be performed while maintaining separation between the hook and loop fastening elements **142, 144,** e.g., with the assistance of vacuum. The hook and loop elements are connected to each other by pressure rollers **434** to form the final refastenable articles **100.** The refastenable articles **100** are thus closed to the "pre-fastened" condition in which the refastenable article **100** is packaged and sold.

### Apparatus for Manufacturing Refastenable Training Pants

Apparatuses for manufacturing a refastenable article are also disclosed. In one aspect, the apparatus has a first side panel attachment device adapted to attach a first side panel having a first refastenable fastening element to the first lateral edge of a pant chassis. The apparatus includes a first side panel folding device adapted to receive the first side panel and to fold the first side panel in a direction substantially transverse to a lateral edge of the pant chassis to form a first folded portion. When attached to the pant chassis, the first folded portion is folded inwardly over the pant chassis and extends between the first and second lateral edges of the pant chassis. The apparatus further includes a securing device adapted to secure the first folded portion of the first side panel to the pant chassis.

In some embodiments, the apparatus may include a first transport device for transporting the pant chassis to the first side panel attachment device. The first transport device may be adapted to transport the pant chassis as a continuous web of topsheet material.

In some embodiments, the apparatus may include a second side panel attachment device that may be adapted to attach a second side panel having a second refastenable fastening element to the pant chassis adjacent to the first lateral edge of the pant chassis. The apparatus may also include a second side panel folding device that is adapted to receive the second side panel and to fold the second side panel in a second direction substantially transverse to a second lateral edge of the pant chassis to form a second folded portion. When attached to the pant chassis, the second folded portion may be folded inwardly over the pant chassis and extend between the first and second lateral edges of the pant chassis. In certain embodiments, the apparatus may further include a second breakable bond forming device that is adapted to form a second breakable bond **36** between the second folded portion of the second side panel and the pant chassis.

In some embodiments, the apparatus may include a third side panel folding device adapted to receive the second side panel and fold the side panel in a third direction substantially transverse to the second waist edge to form a third folded portion, wherein the third folded portion may be folded outwardly over the second folded portion.

In some embodiments, the apparatus may further include a third breakable bond forming device that may be adapted to form a third breakable bond **38** between the third folded portion of the second side panel and the pant chassis. In some embodiments, the apparatus may also include a fourth breakable bond forming device that may be adapted to form a fourth breakable bond **39** between the third folded portion of the second side panel and the pant chassis. In some embodiments, the third breakable **38** bond may be formed between a first tabbed portion **74** of the third folded portion of each of the second pair of side panels and the pant chassis, where the first tabbed portion **74** extends from one of a trailing edge or a leading edge of a distal region of the third folded portion. In some embodiments, the fourth breakable bond **39** may be formed between a second tabbed portion **74** of the third folded portion of each of the second pair of side panels and the pant chassis, where the second tabbed portion **74** extends from another of a trailing edge or a leading edge of the distal region of the third folded portion.

In still other embodiments, a third breakable bond forming device may be adapted to form a third breakable bond between the third folded portion of each of the second side panels and the respective second folded portion of each of the second side panels between the first and second lateral edges of the pant chassis.

In some embodiments, the apparatus may further include a second transport device adapted to transport a continuous web of backsheet material to join with the continuous web of topsheet material after the continuous web of topsheet of material has the first and the second side panels affixed thereto. The apparatus may also include an attachment device for fixing the continuous web of backsheet material to the continuous web of topsheet material.

The apparatus may include a bifolder device that is configured to fold the pant chassis, the first side panels, and the second side panels about a central fold line substantially parallel to the first and second waist edges such that the first refastenable fastening elements and second refastenable fastening elements face each other between the first and second lateral edges of the pant chassis. In certain embodiments, the apparatus may further include a fastening station that is configured to compress the pant chassis and thereby refastenably engage the first and second refastenable fastening elements while the refastenable fastening elements are between the first and second lateral edges of the pant chassis.

In another aspect, an apparatus is provided for preparing a refastenable training pant for packaging. The apparatus may include a pant assembly system configured to prepare a refastenable training pant that includes a chassis having a leading edge, a trailing edge, and first and second lateral edges extending in a longitudinal direction between the leading edge and the trailing edge. The refastenable training pant may also include a first pair of side panels attached to the chassis proximal to one of the leading edge and the trailing edge of the chassis and a second pair of side panels attached to the chassis proximal to another of the leading edge and the trailing edge of the chassis. The first pair of side panels may each include a first fastening component and the second pair of side panels may each include a second fastening component. The first pair of side panels may be folded inwardly over the chassis such that the first fastening components lie between the first and second lateral edges of the chassis, and the second pair of side panels may also be folded inwardly over the chassis such that the second fastening components lie between the first and second lateral edges of the chassis. The apparatus may also include a pant transport device that may be adapted to transport the refastenable training pant in a machine direction while the first and second fastening components lay between the first and second lateral edges of the chassis in an unfastened state. The apparatus may further include a fastening station that may be adapted to refastenably engage the first fastening components and second fastening components while the first fastening components and second fastening components lay between the first and second lateral edges of the chassis.

In some embodiments, the apparatus may further include a folding device that may be adapted to fold the refastenable training pant about a transverse fold line such that the first fastening components of the first pair of side panels face the second fastening components of the second pair of side panels between the first and second lateral edges of the chassis.

The apparatus may also include a first side panel attachment device adapted to attach the first pair of side panels to the first and second lateral edges of the chassis. In certain embodiments, the apparatus may further include a first side panel folding device that may be adapted to receive the first pair of side panels and fold each of the first pair of side panels substantially transverse to a lateral edge of the chassis to form a first folded portion. When attached to the chassis, the first folded portion may be folded inwardly over the chassis and extend between the first and second lateral edges of the chassis. The apparatus may also include a breakable bond forming device that is adapted to form a breakable bond between the first folded portions of the first side panels and the chassis.

In some embodiments, the apparatus may also include a second transport device for transporting the chassis to the second side panel attachment device. For example, the second transport device may be adapted to transport the chassis as a continuous web of topsheet material. The apparatus may further include a second side panel attachment device that is adapted to attach the second pair of side panels to the first and second lateral edges of the chassis. The apparatus may also include a second side panel folding device adapted to receive the second pair of side panels and to fold each of the second pair of side panels substantially transverse to a second waist edge to form a second folded portion, wherein when attached to the chassis the second folded portion is folded inwardly over the chassis and extends between the first and second lateral edges of the chassis. In certain embodiments, the apparatus may further include a second breakable bond forming device that may be adapted to form a second breakable bond **36** between the second folded portions of the second side panels and the chassis.

In some embodiments, the apparatus may further include a third side panel folding device adapted to receive the second pair of side panels and fold each of the second pair of side panels substantially transverse to the second waist edge to form a third folded portion, wherein when attached to the chassis the third folded portion may be folded outwardly over the second folded portion. The apparatus may also include a third breakable bond forming device adapted to form a third breakable bond **38** between the third folded portion of each of the second pair of side panels and the chassis. In some embodiments, the apparatus may also include a fourth breakable bond forming device that may be adapted to form a fourth breakable bond **39** between the third folded portion of the second side panel and the chassis. In some embodiments, the third breakable bond **38** may be formed between a first tabbed portion **74** of the third folded portion of each of the second pair of side panels and the chassis, where the first tabbed portion **74** extends from one of a trailing edge or a leading edge of a distal region of the third folded portion. In some embodiments, the fourth breakable bond **39** may be formed between a second tabbed portion **74** of the third folded portion of each of the second pair of side panels and the chassis, where the second tabbed portion **74** extends from another of a trailing edge or a leading edge of the distal region of the third folded portion.

In still other embodiments, a third breakable bond may be formed between the third folded portion of each of the second side panels and the respective second folded portion of each of the second side panels between the first and second lateral edges of the chassis.

In certain embodiments, the apparatus may include a third transport device that may be adapted to transport a continuous web of backsheet material to join with the continuous web of topsheet material after the continuous web of topsheet of material has the first and the second side panels affixed thereto. In such embodiments, the apparatus may further include an attachment device for fixing the continuous web of backsheet material to the continuous web of topsheet material.

FIG. 12 schematically illustrates an apparatus that is adapted for producing training pants as exemplified in FIG. 3. In this example, the front side panels are attached to the chassis before the back side panels are attached to the chassis. It should be noted, however, that the back side panels may be attached to the chassis either before or after the front side panels are attached to the chassis.

A first side panel attachment device **304** is provided for attaching the front side panels **24** to the pant chassis. The front side panels **24** may be fed to the first side panel attachment device **304** as continuous web materials **302.** (An embodiment of an apparatus for making the side panels **26** is illustrated in FIG. 13 which is described in detail hereinabove.) The continuous web materials **302** may include the fastening element **44** of the refastenable fastening system **40.** The front side panels **24** may then be cut from the continuous web material **302** and then may be applied on the topsheet **16,** which may be fed to the first side panel attachment device **304** as a continuous web material **300.** The first side panel attachment device **304** may function to permanently bond the front side panels **24** to the topsheet **16.** For example, the first side panel attachment device **304** may include an adhesive application device for applying a permanent adhesive to the topsheet **16** and/or front side panels **24** or may include a device for thermally or ultrasonically bonding the front side panels **24** to the topsheet **16.**

A first side panel folding device **306** may be provided for folding the front side panels **24** over topsheet **16** to form the first folded portions **24a.** The first side panel folding device **306** may include, for example, a folding board or a folding conveyor. The first side panel folding device **306** may be positioned upstream or downstream relative to the second side panel attachment device **312** or the first side panel folding device **306** may be incorporated into the first side panel attachment device **304.**

A first securing device **308** may be provided for forming the first breakable bonds **30.** In some embodiments, the first securing device **308** may be a breakable bond forming device. In embodiments in which the breakable bond is formed by thermal or ultrasonic bonding, a thermal or ultrasonic bonding device may be employed to form a thermal or ultrasonic bond to form the first breakable bonds **30.** In embodiments where an adhesive is used to form the first breakable bonds **30,** the first securing device **308** may be located upstream of the first side panel folding device **306.**

A second side panel attachment device **312** may be provided for attaching the back side panels **26** to the pant chassis. The back side panels **26** may be fed to the second side panel attachment device **312** as two continuous web materials **310.** The continuous web materials **310** may include the fastening element **42** of the refastenable fastening system **40.** The back side panels **26** may then cut from the continuous web material **310** and then applied on the topsheet **16,** which may be fed to the second side panel attachment device **312** as a continuous web material **310.** The second side panel attachment device **312** may include a device for permanently bonding the back side panels **26** to the topsheet **16.** For example, the side panel attachment device **312** may include an adhesive application device for applying a permanent adhesive to the topsheet **16** and/or back side panels **26** or may include a device for thermally or ultrasonically bonding the back side panels **26** to the topsheet **16**.

A second side panel folding device **314** may be provided for folding the back side panels **24** over the topsheet **16** to form the second folded portions **26a.** The second side panel folding device **314** may include, for example, a folding board or a folding conveyor. The second side panel folding device **314** may be positioned upstream or downstream relative to the first side panel attachment device **304** or the first side panel folding device **306** may be incorporated into the first side panel attachment device **304.**

A second securing device **316** may be provided for forming the second breakable bonds **36.** In embodiments in which the breakable bond is formed by thermal or ultrasonic bonding, a thermal or ultrasonic bonding device may be employed to form a thermal or ultrasonic bond to form the second breakable bonds **36.** In embodiments where an adhesive is used to form the second breakable bonds **36,** the second securing device **316** may be located upstream of the second side panel folding device **314.**

A third side panel folding device **318** may be provided for folding the back side panels **26** along the third folding line **34** to form the third folded portions **26b.** The third side panel folding device **318** may include, for example, a folding board or a folding conveyor. The third side panel folding device **318** may be positioned upstream or downstream relative to the first side panel attachment device **304** or the third side panel folding device **318** may be incorporated into the first side panel attachment device **304.**

A third securing device **320** may be provided for forming the third and fourth breakable bonds **38, 39.** In some embodiments, separate securing devices may be provided for forming each of the third breakable bonds **38** and the fourth breakable bonds **39**. In embodiments in which the breakable bonds are formed by thermal or ultrasonic bonding, thermal or ultrasonic bonding devices may be employed to form a thermal or ultrasonic bond to form the third and fourth breakable bonds **38, 39.** In embodiments where an adhesive is used to form the third and/or fourth breakable bonds **38, 39,** the third securing device **320** may be located upstream of the third side panel folding device **318.**

An elastic web band attachment unit **322** may be provided for attaching the elastic web bands **48** to the topsheet **16.** The elastic web band attachment unit **322** may attach the elastic web bands **48** to the topsheet with an adhesive or by thermal or ultrasonic bonding.

In a separate feed line, the elastic leg members **50** may be joined to the backsheet **20,** which may be fed as a continuous web material **324.** Absorbent cores **22** from a source **326** may then applied to the backsheet **20.** The topsheet **16** (with the front and back side panels **24, 26** and elastic waist band **48**) may then be superposed and fixed to the backsheet **20** (with the elastic leg members **50** and absorbent cores **22**). The topsheet **16** and backsheet **20** may be fixed together by any process known in the art including adhesive, ultrasonic, or thermal bonding.

In some embodiments, a leg cuff attachment unit may be provided for attaching the leg cuffs 21 to the top sheet 16. The leg cuff attachment unit may attach the leg cuffs 21 to the top sheet 16 with an adhesive or by thermal or ultrasonic bonding.

A cutting station 328 may be provided for transversally cutting the continuous webs to form blanks of refastenable articles 10. The cut refastenable articles 10 may then be fed to a roller 330. The refastenable articles 10 may be maintained on the outer surface of a roller 330 by vacuum.

A folder 332, which may be referred to as a bifolder, folds the refastenable articles 10 along respective transverse folding lines A as the crotch region 29 of the refastenable article 10 may be fed through a folding nip which may include a pair of nip rollers. The roller 330 may include a pusher for ejecting the refastenable article 10 from the folder into the folding nip.

The folder may include a pair of conveyors, for example, top and bottom conveyors in the present illustration, which pull the refastenable article 10 through the folding nip and transport the refastenable article 10 downstream of the folding nip. In some embodiments, the folder 332 may maintain the refastenable articles HI in position against the conveyors during folding by vacuum. For example, the folder 332 may include vacuum nip rolls at the folding nip and/or Vacuum conveyors. In some embodiments, the conveyors may each include a belt with areas for gripping the refastenable articles 10. The gripping areas may be formed by a plurality of seats that are recessed with respect to the outer surfaces of the belts. The gripping areas for picking up refastenable articles 10 are provided with holes for picking up the refastenable articles 10 by vacuum suction. The arrangement of holes may be such as to reproduce the shapes of the respective front half and rear half of the refastenable article 10. The surfaces of the top and bottom belts may be sufficiently close so as to compress between them the side panels 24, 26. This solution enables fastening of the refastenable article 10 directly during the step of folding. Further details about the conveyors may be found in U.S. Provisional Patent Application Serial No. 61/453,677, filed on March 17, 2011 and published as US 2014/011655.

In other embodiments, the folder 332 may fold the refastenable articles 10 without the assistance of vacuum. Advantageously, it has been found that commercial production speeds may be achieved without the use of vacuum because of the stability provided by the breakable bonds to the side panels.

When folding the refastenable article **10** in the folder **332,** the refastening elements **42, 44** of the refastenable fastening system **40** and the front and back side panels **24, 26** may be maintained within the chassis between the folded portions **24a, 26a,** and **26b** and the lateral edges **14** by detachable securement such that the folded portions **24a, 26a,** and **26b** of the side panels **24** and **26** are disposed inwardly with respect to the outer perimeter of the chassis **12.**

In some embodiments, the transversal folding operation performed in the folder **332** may bring the hook and loop fastening elements **42, 44** of the refastenable fastening system **40** into contact with each other. In other embodiments, the transversal folding operation performed by the folder **332** may be performed while maintaining separation between the hook and loop fastening elements **42, 44,** e.g., with the assistance of vacuum. The hook and loop elements may be connected to each other by pressure rollers **334** to form the final refastenable articles **10.** The refastenable articles **10** may thus be closed to the "pre-fastened" condition in which the refastenable article **10** is packaged and sold.

FIG. 15 schematically illustrates an apparatus that is adapted for producing training pants as exemplified in FIGS. 6-9. In this example, the first side panels are attached to the chassis before the second panels are attached to the chassis. It should be noted, however, that the first side panels may be attached to the chassis either before or after the second side panels are attached to the chassis.

A first side panel attachment device **404** is provided for attaching the front side panels **124** to the pant chassis. The front side panels **124** may be fed to the first side panel attachment device **404** as continuous web materials **402.** The continuous web materials **402** may include the fastening element **144** of the refastenable fastening system **140.** The front side panels **124** may then be cut from the continuous web material **402** and then may be applied on the topsheet **116,** which is fed to the first side panel attachment device **404** as a continuous web material **400.** The first side panel attachment device **404** may include a device for permanently bonding the front side panels **124** to the topsheet **116.** For example, the first side panel attachment device **404** may include an adhesive application device for applying a permanent adhesive to the topsheet **116** and/or front side panels **124** or may include a device for thermally or ultrasonically bonding the front side panels **124** to the topsheet **116.**

A leg cuff attachment unit **405** may be provided for attaching the leg cuffs **121** to the topsheet **116.** The leg cuff attachment unit **405** may attach the leg cuffs **121** to the topsheet **116** with an adhesive or by thermal or ultrasonic bonding. The leg cuffs **121** may be configured to create pockets **180** adjacent to the corners of the topsheet **116.** The pockets **180** may be used to at least partially secure the folded front and back side panels **124, 126** such that the transversal folding operation performed in the folder **432** will bring the hook and loop fastening elements **142, 144** of the refastenable fastening system **140** into contact with each other.

A first side panel folding device **406** may be provided for folding the front side panels **124** over the topsheet **116** to form the first folded portions **124a.** The first side panel folding device **406** may include, for example, a folding board or a folding conveyor. The first side panel folding device **406** may be incorporated into the first side panel attachment device **404.**

A first securing device **416** may be provided for at least partially inserting (i.e., tucking) the folded front side panels **124** into respective pockets **180** that may be located adjacent to each of the first and second lateral edges of the chassis. In some embodiments, the first securing device **416** may comprise a tucking device. In some embodiments, the first securing device **416** may at least partially insert the folded front side panels **124** into respective pockets **180** that are formed between the barrier leg cuffs **121** and the topsheet **116.**

In still other embodiments, the first side panel folding device **406** may fold the front side panels **124** over the topsheet **116** to form the first folded portions **124a.** The leg cuff attachment unit **405** may then attach the leg cuffs **121** to the topsheet **116.** The leg cuffs **121** may be configured to create pockets **180** adjacent to the corners of the topsheet **116.** The pockets **180** may be used to at least partially secure the folded front side panels **124.**

A second side panel attachment device **412** may be provided for attaching the back side panels **126** to the pant chassis. The back side panels **126** may be fed to the second side panel attachment device **412** as two continuous web materials **410.** The continuous web materials **410** may include the fastening element **142** of the refastenable fastening system **140.** The back side panels **126** may then be cut from the continuous web material **410** and may then applied on the topsheet **116,** which may be fed to the second side panel attachment device **412** as a continuous web material **410.**

A second side panel folding device **414** may be provided for folding the back side panels **126** over the topsheet **116** to form the second folded portions **126a.** The second side panel folding device **414** may include, for example, a folding board or a folding conveyor. The second side panel folding device **414** may be positioned upstream or downstream relative to the first side panel attachment device **404** or the second side panel folding device **414** may be incorporated into the first side panel attachment device **404.**

A third side panel folding device **418** may be provided for folding the back side panels **126** along the third folding line **134** to form the third folded portions **126b.** The third side panel folding device **418** may include, for example, a folding board or a folding conveyor. The third side panel folding device **418** may be positioned upstream or downstream relative to the first side panel attachment device **404** or the third side panel folding device **418** may be incorporated into the first side panel attachment device **404.**

A second securing device **420** may be provided for at least partially inserting (i.e., tucking) the folded back side panels **126** into respective pockets **180** that may be located adjacent to each of the first and second lateral edges of the chassis. In some embodiments, the second securing device **420** may at least partially insert the folded back side panels **126** into respective pockets **180** that are formed between the barrier leg cuffs **121** and the topsheet **116.**

In still other embodiments, the first side panel folding device **406** may fold the front side panels **124** over the topsheet **116** to form the first folded portions **124a.** The second side panel folding device **414** may be provided for folding the back side panels **126** over the topsheet **116** to form the second folded portions **126a.** The third side panel folding device **418** may be provided for folding the back side panels **126** along the third folding line **134** to form the third folded portions **126b.** The leg cuff attachment unit **405** may then attach the leg cuffs **121** to the topsheet **116.** The leg cuffs **121** may be configured to create pockets **180** adjacent to the corners of the topsheet **116.** The pockets **180** may be used to at least partially secure the folded front and back side panels **124,126.**

An elastic web band attachment unit **422** may be provided for attaching the elastic web bands **148** to the topsheet **116.** The elastic web band attachment unit **422** may attach the elastic web bands **148** to the topsheet **116** with an adhesive or by thermal or ultrasonic bonding.

In a separate feed line, the elastic leg members **150** may be joined to the backsheet **120,** which may be fed as a continuous web material **424.** Absorbent cores **122** from a source **426** may then be applied to the backsheet **120.** The topsheet **116** (with the front and back side panels **124, 126** and elastic waist band **148**) may then be superposed and fixed to the backsheet **120** (with the elastic leg members **150** and absorbent cores **122**). The topsheet **116** and backsheet **120** may be fixed together by any process known in the art including adhesive, ultrasonic, or thermal bonding.

A cutting station **428** may be provided for transversally cutting the continuous webs to form blanks of refastenable articles **100.** The cut refastenable articles **100** may then be fed to a roller **430.** The refastenable articles **10** may be maintained on the outer surface of a roller **430** by vacuum.

A folder **432,** which may be referred to as a bifolder, may fold the refastenable articles **100** along respective transverse folding lines A as the crotch region **129** of the refastenable article **100** is fed through a folding nip which includes a pair of nip rollers. The roller **430** may include a pusher for ejecting the refastenable article **100** from the folder into the folding nip. The folder may include a pair of conveyors, for example, top and bottom conveyors in the present illustration, which may pull the refastenable article **100** through the folding nip and transport the refastenable article **100** downstream of the folding nip. In some embodiments, the folder **432** may maintain the refastenable articles **100** in position against the conveyors during folding by vacuum. Additional details of the bifolder are the same as those described hereinabove. For example, the folder **432** may include vacuum nip rolls at the folding nip and/or vacuum conveyors. In other embodiments, the folder **432** may fold the refastenable articles **100** without the assistance of vacuum.

When folding the refastenable article **100** in the folder **432,** the refastening elements **142, 144** of the refastenable fastening system **140** and the front and back side panels **124, 126** may be maintained within the chassis between the folded portions **124a, 126a,** and **126b** and the lateral edges **114** by the respective pockets **180** such that the folded portions 124a, 126a, and 126b of the side panels 124 and 126 are disposed inwardly with respect to the outer perimeter of the chassis 112.

In some embodiments, the transversal folding operation performed in the folder 432 may bring the hook and loop fastening elements 142, 144 of the refastenable fastening system 140 into contact with each other. In other embodiments, the transversal folding operation may be performed by the folder 432 and may be performed while maintaining separation between the hook and loop fastening elements 142, 144, e.g., with the assistance of vacuum. The hook and loop elements may be connected to each other by pressure rollers 434 to form the final refastenable articles 100. The refastenable articles 100 may thus be closed to the "pre-fastened" condition In which the training pant refastenable article 100 may be packaged and sold.

While adhesively connected elements have been considered by way of example, various embodiments may adopt connection via thermal bonding, ultrasound bonding, gluing, or combinations of the various techniques considered.

Those who are skilled in the art will promptly appreciate that the representations of FIGs. 12 and 15 are by their nature schematic. Other features of the apparatus and system of FIGs. 12 and 15 not specifically considered herein are conventional in the art, thus making it unnecessary to provide detailed description herein.

## Claims

1. A method of making a refastenable article (10, 100) comprising:
providing a refastenable training pant (10, 100) comprising a chassis (12, 112) having a leading edge (41, 141), a trailing edge (43, 143), and first and second lateral edges (14, 114) extending in a longitudinal direction between the leading edge (41, 141) and the trailing edge (43, 143), the refastenable training pant (10, 100) comprising a first pair of side panels (24, 124) attached to the chassis (12, 112) proximal one of the leading edge (41, 141) and trailing edge (43, 143) of the chassis (12, 112) and a second pair of side panels (26, 126) attached to the chassis (12, 112) proximal another of the leading edge (41, 141) and the trailing edge (43, 143) of the chassis (12, 112), wherein the first pair of side panels (24, 124) each comprise a first fastening component (42, 142) and the second pair of side panels (26, 126) each comprise a second fastening component (44, 144); wherein the first pair of side panels (24, 124) are folded inwardly over the chassis (12, 112) such that the first fastening components (42, 142) lie between the first and second lateral edges (14, 114), and wherein the second pair of side panels (26, 126) are folded inwardly over the chassis (12, 112) such that the second fastening components (44, 144) lie between the first and second lateral edges (14, 114) of the chassis (12, 112);
transporting the refastenable training pant (10, 100) in a machine direction through a folding device (332, 432), wherein the refastenable training pant (10, 100) is transported through the folding device (332, 432) with the first pair of side panels (24, 124) folded inwardly over the chassis (12, 112) such that the first fastening components (42, 142) lie between the first and second lateral edges (14, 114), and the second pair of side panels (26, 126) folded inwardly over the chassis (12, 112) such that the second fastening components (26, 126) lie between the first and second lateral edges (14, 114) of the chassis (12, 112);
folding the refastenable training pant (10, 100) about a transverse fold line A with the folding device (332, 432) such that the first fastening components (42, 142) of the first pair of side panels (24, 124) face the second fastening components (44, 144) of the second pair of side panels (26, 126) between the first and second lateral edges (14, 114) of the chassis (12, 112);
refastenably engaging at least a portion of the first fastening components (42, 142) and at least a portion of the second fastening components (44, 144) while the first fastening components (42, 142) and second fastening components (44, 144) lie between the first and second lateral edges (14, 114) of the chassis (12, 112);
the method further comprising folding each of the first pair of side panels (26, 126) inwardly over the chassis (12, 112) such that a first folded portion (24a, 124a) of each of the first side panels (24, 124) and the first fastening components (42, 142) lie between the first and second lateral edges (14, 114) of the chassis (12, 112); and
detachably securing the first folded portion (24a, 124a) of each of the pair of first side panels (24, 124) to the chassis (12, 112).

2. The method of claim 1, further comprising at least partially inserting the first folded portion (24a, 124a) of each of the first pair of side panels (24, 124) into respective pockets (180) located adjacent to each of the first and second lateral edges (14, 114) of the chassis (12, 112).

3. The method of claim 2, wherein the pockets (180) are formed between a leg cuff (121) and a topsheet (116).

4. The method of claim 1, further comprising folding each of the second pair of side panels (26, 126) inwardly over the chassis (12, 112) such that a second folded portion (26a, 126a) of each of the second side panels (26, 126) lies between the first and second lateral edges (14, 114) of the chassis (12, 112); and
folding each of the second pair of side panels (26, 126) outwardly over the second folded portion (26a, 126a) of each side panel such that a third folded portion (26b, 126b) of each of the second pair of side panels (26, 126) lies between the first and second lateral edges (14, 114) of the chassis (12, 112).

5. The method of claim 4, further comprising at least partially inserting the second (26a, 126a) and third (26b, 126b) folded portions of each of the second pair of side panels (26, 126) into respective pockets (180) located adjacent to each of the first and second lateral edges (14, 114) of the chassis (12, 112).

6. A refastenable training pant (10, 100) comprising
a chassis (12, 112) having a leading edge (41, 141), a trailing edge (43, 143), and first and second lateral edges (14, 114) extending in a longitudinal direction between the leading edge (41, 141) and the trailing edge (43, 143), wherein the chassis (12, 112) is folded-over along a transverse fold line orthogonal to the first and second lateral edges 14, 114);
a pair of first side panels (24, 124) attached to the chassis (12, 112) proximal one of the leading edge (41, 141) and the trailing edge (43, 143) of the chassis (12, 112), each of the pair of first side panels (24, 124) comprising a first fastening component (42, 142); and
a pair of second side panels (26, 126) attached to the chassis (12, 112) proximal another of the leading edge (41, 141) and the trailing edge (43, 143) of the chassis (12, 112), each of the pair of second side panels (26, 126) comprising second fastening component 44, 144); wherein
the first fastening components (24, 124) and second fastening components (26, 126) are refastenably engaged between the first and second lateral edges (14, 114) of the chassis (12, 112);
wherein each of the pair of first side panels (24, 124) is folded inwardly over the chassis (12, 112) such that a first folded portion (24a, 124a) of each of the first side panels (24, 124) lies between the first and second lateral edges (14, 114) of the chassis (12, 112);
wherein the first folded portion (24a, 124a) of each of the pair of first side panels (24, 124) is detachably secured to the chassis (12, 112); and
wherein each of the pair of second side panels (26, 126) is folded inwardly over the chassis (12, 112) such that a second folded portion (26a, 126a) of each of the second side panels (26, 126) lies between the first and second lateral edges (14, 114) of the chassis (12, 112).

7. The refastenable training pant (10, 100) of claim 6, wherein a first folded portion (24a, 124a) of each of the pair of first side panels (24, 124) is detachably secured to the chassis (12, 112) by at least partially inserting the first side panels (24, 124) into respective pockets (180);
wherein each of the pair of second side panels (26, 126) is folded inwardly over the chassis (12, 112) such that a second folded portion (26a, 126a) of each of the second side panels (26, 126) lies between the first and second lateral edges (14, 114) of the chassis (12, 112); and
wherein each of the pair of second side panels (26, 126) is at least partially inserted into a respective pocket (180).

8. The refastenable training pant (10, 100) of claim 7, wherein the pockets (180) are formed between a leg cuff (121) and a topsheet (116).

9. The refastenable training pant (10, 100) of claim 6, wherein the first side panels (24, 124) have respective lower edges (146) shaped to anatomically conform to the legs of a wearer.

10. The refastenable training pant (10, 100) of claim 6, wherein the one of the first (42, 142) and second (44, 144) fastening components comprises a loop element formed by surface loops of a material constituting the corresponding side panel.

## Patentansprüche

1. Verfahren zur Herstellung eines wiederverschließbaren Artikels (10, 100), umfassend:
Bereitstellen einer wiederverschließbaren Trainingshose (10, 100), umfassend einen Hauptteil (12, 112) mit einer Vorderkante (41, 141), einer Hinterkante (43, 143) und einer ersten und einer zweiten Seitenkante (14, 114), die sich in Längsrichtung zwischen der Vorderkante (41, 141) und der Hinterkante (43, 143) erstrecken, wobei die wiederverschließbare Trainingshose (10, 100) ein erstes Paar Seitenteile (24, 124), die am Hauptteil (12, 112) nahe der Vorderkante (41, 141) und der Hinterkante (43, 143) des Hauptteils (12, 112) angebracht sind, und ein zweites Paar Seitenteile (26, 126) umfasst, die nahe dem jeweils anderen von Vorderkante (41, 141) und Hinterkante (43, 143) des Hauptteils (12, 112) am Hauptteil (12, 112) angebracht sind, wobei das erste Paar Seitenteile (24, 124) je eine erste Befestigungskomponente (42, 142) umfasst und das zweite Paar Seitenteile (26, 126) je eine zweite Befestigungskomponente (44, 144) umfasst; wobei das erste Paar Seitenteile (24, 124) nach innen über den Hauptteil (12, 112) gefaltet wird, so dass die ersten Befestigungskomponenten (42, 142) zwischen der ersten und der zweiten Seitenkante (14, 114) liegen, und wobei das zweite Paar Seitenteile (26, 126) nach innen über den Hauptteil (12, 112) gefaltet wird, so dass die zweiten Befestigungskomponenten (44, 144) zwischen der ersten und der zweiten Seitenkante (14, 114) des Hauptteils (12, 112) liegen;
Transportieren der wiederverschließbaren Trainingshose (10, 100) in einer Bearbeitungsrichtung durch eine Faltvorrichtung (332, 432), wobei die wiederverschließbare Trainingshose (10, 100) durch die Faltvorrichtung (332, 432) transportiert wird, mit dem ersten Paar Seitenteile (24, 124) nach innen über den Hauptteil (12, 112) gefaltet, so dass die ersten Befestigungskomponenten (42, 142) zwischen der ersten und der zweiten Seitenkante (14, 114) liegen, und dem zweiten Paar Seitenteile (26, 126) nach innen über den Hauptteil (12, 112) gefaltet, so dass die zweiten Befestigungskomponenten (26, 126) zwischen der ersten und der zweiten Seitenkante (14, 114) des Hauptteils (12, 112) liegen;
Falten der wiederverschließbaren Trainingshose (10, 100) über eine querlaufende Faltlinie A mit der Faltvorrichtung (332, 432), so dass die ersten Befestigungskomponenten (42, 142) des ersten Paares Seitenteile (24, 124) zu den zweiten Befestigungskomponenten (44, 144) des zweiten Paares Seitenteile (26, 126) zwischen der ersten und der zweiten Seitenkante (14, 114) des Hauptteils (12, 112) hin weisen;
Ineingriffbringen, in wiederverschließbarer Weise, wenigstens eines Teils der ersten Befestigungskomponenten (42, 142) und wenigstens eines Teils der zweiten Befestigungskomponenten (44, 144), während die ersten Befestigungskomponenten (42, 142) und die zweiten Befestigungskomponenten (44, 144) zwischen der ersten und der zweiten Seitenkante (14, 114) des Hauptteils (12, 112) liegen;
wobei das Verfahren ferner umfasst, jeden Teil des ersten Paares Seitenteile (26, 126) nach innen über den Hauptteil (12, 112) zu falten, so dass ein erster gefalteter Teil (24a, 124a) jedes der ersten Seitenteile (24, 124) und die ersten Befestigungskomponenten (42, 142) zwischen der ersten und der zweiten Seitenkante (14, 114) des Hauptteils (12, 112) liegen; und
Befestigen, in lösbarer Weise, des ersten gefalteten Teils (24a, 124a) jedes Teils des Paares erster Seitenteile (24, 124) am Hauptteil (12, 112).

2. Verfahren nach Anspruch 1, ferner umfassend, wenigstens teilweise den ersten gefalteten Teil (24a, 124a) jedes Teils des ersten Paares Seitenteile (24, 124) in entsprechende Taschen (180) einzustecken, die angrenzend jeweils an die erste und an die zweite Seitenkante (14, 114) des Hauptteils (12, 112) angeordnet sind.

3. Verfahren nach Anspruch 2, wobei die Taschen (180) zwischen einem Beinbund (121) und einer Deckschicht (116) ausgebildet werden.

4. Verfahren nach Anspruch 1, ferner umfassend, jeden Teil des zweiten Paares Seitenteile (26, 126) nach innen über den Hauptteil (12, 112) zu falten, so dass ein zweiter gefalteter Teil (26a, 126a) jedes der zweiten Seitenteile (26, 126) zwischen der ersten und der zweiten Seitenkante (14, 114) des Hauptteils (12, 112) liegt; und
Falten jedes Teils des zweiten Paares Seitenteile (26, 126) nach außen über den zweiten gefalteten Teil (26a, 126a) jedes Seitenteils, so dass ein dritter gefalteter Teil (26b, 126b) jedes Teils des zweiten Paares Seitenteile (26, 126) zwischen der ersten und der zweiten Seitenkante (14, 114) des Hauptteils (12, 112) liegt.

5. Verfahren nach Anspruch 4, ferner umfassend, wenigstens teilweise den zweiten (26a, 126a) und den dritten (26b, 126b) gefalteten Teil jedes Teils des zweiten Paares Seitenteile (26, 126) in entsprechende Taschen (180) einzustecken, die angrenzend jeweils an die erste und an die zweite Seitenkante (14, 114) des Hauptteils (12, 112) angeordnet sind.

6. Wiederverschließbare Trainingshose (10, 100), umfassend:
einen Hauptteil (12, 112) mit einer Vorderkante (41, 141), einer Hinterkante (43, 143) und einer ersten und einer zweiten Seitenkante (14, 114), die sich in Längsrichtung zwischen der Vorderkante (41, 141) und der Hinterkante (43, 143) erstrecken, wobei der Hauptteil (12, 112) entlang einer querlaufenden Faltlinie rechtwinklig zur ersten und zur zweiten Seitenkante 14, 114) gefaltet wird; ein Paar erster Seitenteile (24, 124), die am Hauptteil (12, 112) nahe der Vorderkante (41, 141) bzw. der Hinterkante (43, 143) des Hauptteils (12, 112) angebracht sind, wobei jeder Teil des Paares erster Seitenteile (24, 124) eine erste Befestigungskomponente (42, 142) umfasst; und ein Paar zweiter Seitenteile (26, 126), die nahe dem jeweils anderen von Vorderkante (41, 141) bzw. Hinterkante (43, 143) des Hauptteils (12, 112) am Hauptteil (12, 112) angebracht sind, wobei jeder Teil des Paares zweiter Seitenteile (26, 126) eine zweite Befestigungskomponente (44, 144) umfasst; wobei
die ersten Befestigungskomponenten (24, 124) und die zweiten Befestigungskomponenten (26, 126) in wiederverschließbarer Weise zwischen der ersten und der zweiten Seitenkante (14, 114) des Hauptteils (12, 112) in Eingriff sind;
wobei jeder Teil des Paares erster Seitenteile (24, 124) nach innen über den Hauptteil (12, 112) gefaltet wird, so dass ein erster gefalteter Teil (24a, 124a) jedes der ersten Seitenteile (24, 124) zwischen der ersten und der zweiten Seitenkante (14, 114) des Hauptteils (12, 112) liegt;
wobei der erste gefaltete Teil (24a, 124a) jedes Teils des Paares erster Seitenteile (24, 124) in lösbarer Weise am Hauptteil (12, 112) befestigt ist; und
wobei jeder Teil des zweiten Paares Seitenteile (26, 126) nach innen über den Hauptteil (12, 112) gefaltet wird, so dass ein zweiter gefalteter Teil (26a, 126a) jedes der zweiten Seitenteile (26, 126) zwischen der ersten und der zweiten Seitenkante (14, 114) des Hauptteils (12, 112) liegt.

7. Wiederverschließbare Trainingshose (10, 100) nach Anspruch 6, wobei ein erster gefalteter Teil (24a, 124a) jedes Teils des Paares erster Seitenteile (24, 124) in lösbarer Weise am Hauptteil (12, 112) befestigt wird, indem wenigstens die ersten Seitenteile (24, 124) in entsprechende Taschen (180) eingesteckt werden;
wobei jeder Teil des zweiten Paares Seitenteile (26, 126) nach innen über den Hauptteil (12, 112) gefaltet wird, so dass ein zweiter gefalteter Teil (26a, 126a) jedes der zweiten Seitenteile (26, 126) zwischen der ersten und der zweiten Seitenkante (14, 114) des Hauptteils (12, 112) liegt; und
wobei jeder Teil des Paares zweiter Seitenteile (26, 126) wenigstens teilweise in eine entsprechende Tasche (180) eingesteckt wird.

8. Wiederverschließbare Trainingshose (10, 100) nach Anspruch 7, wobei die Taschen (180) zwischen einem Beinbund (121) und einer Deckschicht (116) ausgebildet werden.

9. Wiederverschließbare Trainingshose (10, 100) nach Anspruch 6, wobei die ersten Seitenteile (24, 124) jeweilige Unterkanten (146) aufweisen, die so ausgeformt sind, dass sie anatomisch an die Beine eines Trägers angepasst sind.

10. Wiederverschließbare Trainingshose (10, 100) nach Anspruch 6, wobei die eine der ersten (42, 142) und zweiten (44, 144) Befestigungskomponenten ein Schlaufenelement aufweist, das durch oberflächliche Schlaufen eines Materials gebildet wird, aus dem der entsprechende Seitenteil besteht.

## Revendications

1. Procédé de fabrication d'un article rattachable (10, 100) comprenant le fait :
de fournir une culotte de propreté rattachable (10, 100) comprenant un châssis (12, 112) ayant un bord avant (41, 141), un bord arrière (43, 143), et des premier et deuxième bords latéraux (14,114) s'étendant dans une direction longitudinale entre le bord avant (41, 141) et le bord arrière (43, 143), la culotte de propreté rattachable (10, 100) comprenant une première paire de panneaux latéraux (24, 124) fixés au châssis (12, 112) à proximité de l'un du bord avant (41, 141) et du bord arrière (43, 143) du châssis (12, 112) et une deuxième paire de panneaux latéraux (26, 126) fixés au châssis (12, 112) à proximité de l'autre du bord avant (41, 141) et du bord arrière (43, 143) du châssis (12, 112), où chacun de la première paire de panneaux latéraux (24, 124) comprend un premier composant d'attache (42, 142) et chacun de la deuxième paire de panneaux latéraux (26, 126) comprend un deuxième composant d'attache (44, 144) ; où les panneaux de la première paire de panneaux latéraux (24, 124) sont pliés vers l'intérieur sur le châssis (12, 112) de sorte que les premiers composants d'attache (42, 142) se trouvent entre les premier et deuxième bords latéraux (14, 114), et où les panneaux de la deuxième paire de panneaux latéraux (26, 126) sont pliés vers l'intérieur sur le châssis (12, 112) de sorte que les deuxièmes composants d'attache (44, 144) se trouvent entre les premier et deuxième bords latéraux (14, 114) du châssis (12, 112) ;
d'acheminer la culotte de propreté rattachable (10, 100) dans un sens machine à travers un dispositif de pliage (332, 432), où la culotte de propreté rattachable (10, 100) est acheminée à travers le dispositif de pliage (332, 432) avec la première paire de panneaux latéraux (24, 124) pliée vers l'intérieur sur le châssis (12, 112) de sorte que les premiers composants d'attache (42, 142) se trouvent entre les premier et deuxième bords latéraux (14, 114), et la deuxième paire de panneaux latéraux (26, 126) pliée vers l'intérieur sur le châssis (12, 112) de sorte que les deuxièmes composants d'attache (26, 126) se trouvent entre les premier et deuxième bords latéraux (14, 114) du châssis (12, 112) ;
de plier la culotte de propreté rattachable (10, 100) autour d'une ligne de pliage transversale A avec le dispositif de pliage (332, 432) de sorte que les premiers composants d'attache (42, 142) de la première paire de panneaux latéraux (24, 124) soient opposés aux deuxièmes composants d'attache (44, 144) de la deuxième paire de panneaux latéraux (26, 126) entre les premier et deuxième bords latéraux (14, 114) du châssis (12, 112) ;
de mettre en prise de manière rattachable au moins une partie des premiers composants d'attache (42, 142) avec au moins une partie des deuxièmes composants d'attache (44, 144) tandis que les premiers composants d'attache (42, 142) et les deuxièmes composants d'attache (44, 144) se trouvent entre les premier et deuxième bords latéraux (14, 114) du châssis (12, 112) ;
le procédé comprenant en outre le fait de plier chacun de la première paire de panneaux latéraux (26, 126) vers l'intérieur sur le châssis (12, 112) de sorte qu'une première partie pliée (24a, 124a) de chacun des premiers panneaux latéraux (24, 124) et des premiers composants d'attache (42, 142) se trouve entre les premier et deuxième bords latéraux (14, 114) du châssis (12, 112) ; et
de fixer de manière amovible la première partie pliée (24a, 124a) de chacun de la paire de premiers panneaux latéraux (24, 124) au châssis (12, 112).

2. Procédé de la revendication 1, comprenant en outre le fait d'insérer au moins partiellement la première partie pliée (24a, 124a) de chacun de la première paire de panneaux latéraux (24, 124) dans des poches respectives (180) situées de manière adjacente à chacun des premier et deuxième bords latéraux (14, 114) du châssis (12, 112).

3. Procédé de la revendication 2, dans lequel les poches (180) sont formées entre un revers de jambe (121) et une feuille supérieure (116).

4. Procédé de la revendication 1, comprenant en outre le fait de plier chacun de la deuxième paire de panneaux latéraux (26, 126) vers l'intérieur sur le châssis (12, 112) de sorte qu'une deuxième partie pliée (26a, 126a) de chacun des deuxièmes panneaux latéraux (26, 126) se trouve entre les premier et deuxième bords latéraux (14, 114) du châssis (12, 112) ; et
de plier chacun de la deuxième paire de panneaux latéraux (26, 126) vers l'extérieur sur la deuxième partie pliée (26a, 126a) de chaque panneau latéral de sorte qu'une troisième partie pliée (26b, 126b) de chacun de la deuxième paire de panneaux latéraux (26, 126) se trouve entre les premier et deuxième bords latéraux (14, 114) du châssis (12, 112).

5. Procédé de la revendication 4, comprenant en outre le fait d'insérer au moins partiellement les deuxième (26a, 126a) et troisième (26b, 126b) parties pliées de chacun de la deuxième paire de panneaux latéraux (26, 126) dans des poches respectives (180) situées de manière adjacente à chacun des premier et deuxième bords latéraux (14, 114) du châssis (12, 112).

6. Culotte de propreté rattachable (10, 100) comprenant
un châssis (12, 112) ayant un bord avant (41, 141), un bord arrière (43, 143), et des premier et deuxième bords latéraux (14, 114) s'étendant dans une direction longitudinale entre le bord avant (41, 141) et le bord arrière (43, 143), où le châssis (12, 112) est replié le long d'une ligne de pliage transversale orthogonale aux premier et deuxième bords latéraux (14, 114) ; une paire de premiers panneaux latéraux (24, 124) fixés au châssis (12, 112) à proximité de l'un du bord avant (41, 141) et du bord arrière (43, 143) du châssis (12, 112), chacun de la paire de premiers panneaux latéraux (24, 124) comprenant un premier composant d'attache (42, 142) ; et une paire de deuxièmes panneaux latéraux (26, 126) fixés au châssis (12, 112) à proximité de l'autre du bord avant (41, 141) et du bord arrière (43, 143) du châssis (12, 112), chacun de la paire de deuxièmes panneaux latéraux (26, 126) comprenant un deuxième composant d'attache (44, 144) ; où
les premiers composants d'attache (24, 124) et les deuxièmes composants d'attache (26, 126) se mettent en prise de manière rattachable entre les premier et deuxième bords latéraux (14, 114) du châssis (12, 112) ;
où chacun de la paire de premiers panneaux latéraux (24, 124) est plié vers l'intérieur sur le châssis (12, 112) de sorte qu'une première partie pliée (24a, 124a) de chacun des premiers panneaux latéraux (24, 124) se trouve entre les premier et deuxième bords latéraux (14, 114) du châssis (12, 112) ;
où la première partie pliée (24a, 124a) de chacun de la paire de premiers panneaux latéraux (24, 124) est fixée de manière amovible au châssis (12, 112) ; et
où chacun de la paire de deuxièmes panneaux latéraux (26, 126) est plié vers l'intérieur sur le châssis (12, 112) de sorte qu'une deuxième partie pliée (26a, 126a) de chacun des deuxièmes panneaux latéraux (26, 126) se trouve entre les premier et deuxième bords latéraux (14, 114) du châssis (12, 112).

7. Culotte de propreté rattachable (10, 100) de la revendication 6, dans laquelle une première partie pliée (24a, 124a) de chacun de la paire de premiers panneaux latéraux (24, 124) est fixée de manière amovible au châssis (12, 112) en insérant au moins partiellement les premiers panneaux latéraux (24, 124) dans des poches respectives (180) ;
où chacun de la paire de deuxièmes panneaux latéraux (26, 126) est plié vers l'intérieur sur le châssis (12, 112) de sorte qu'une deuxième partie pliée (26a, 126a) de chacun des deuxièmes panneaux latéraux (26, 126) se trouve entre les premier et deuxième bords latéraux (14, 114) du châssis (12, 112) ; et
où chacun de la paire de deuxièmes panneaux latéraux (26, 126) est inséré au moins partiellement dans une poche respective (180).

8. Culotte de propreté rattachable (10, 100) de la revendication 7, dans lequel les poches (180) sont formées entre un revers de jambe (121) et une feuille supérieure (116).

9. Culotte de propreté rattachable (10, 100) de la revendication 6, dans laquelle les premiers panneaux latéraux (24, 124) ont des bords inférieurs respectifs (146) façonnés de manière à se conformer anatomiquement aux jambes d'une personne la portant.

10. Culotte de propreté rattachable (10, 100) de la revendication 6, dans laquelle l'un des premier (42, 142) et deuxième (44, 144) composants d'attache comprend un élément en boucle formé par des boucles de surface d'un matériau constituant le panneau latéral correspondant.
